(19)

(11) **EP 3 752 214 B1**

(12) **EUROPEAN PATENT SPECIFICATION**

(45) Date of publication and mention of the grant of the patent:
**05.08.2026 Bulletin 2026/32**

(21) Application number: **19706886.9**

(22) Date of filing: **31.01.2019**

(51) International Patent Classification (IPC):
***A61M 1/14*** *(2006.01)* ***A61M 1/36*** *(2006.01)*
***A61M 1/16*** *(2006.01)*

(52) Cooperative Patent Classification (CPC):
**A61M 1/1601; A61M 1/14; A61M 1/36**

(86) International application number:
**PCT/EP2019/052341**

(87) International publication number:
**WO 2019/158364 (22.08.2019 Gazette 2019/34)**

(54) **FILTERING A PRESSURE SIGNAL FROM A MEDICAL APPARATUS**

FILTERUNG EINES DRUCKSIGNALS AUS EINEM MEDIZINISCHEN GERÄT

FILTRAGE D'UN SIGNAL DE PRESSION PROVENANT D'UN APPAREIL MÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.02.2018 SE 1850175**

(43) Date of publication of application:
**23.12.2020 Bulletin 2020/52**

(73) Proprietor: **Gambro Lundia AB**
**226 43 Lund (SE)**

(72) Inventors:
- **OLDE, Bo**
  **224 67 LUND (SE)**
- **EKDAHL, Olof**
  **226 44 Lund (SE)**
- **VARTIA, Christian**
  **247 64 VEBERÖD (SE)**

(74) Representative: **Sweden SHS IP Office**
**Gambro Lundia AB**
**P.O. Box 10101**
**220 10 Lund (SE)**

(56) References cited:
**WO-A1-2012/170119**
**WO-A1-2013/000777**
**WO-A1-2014/035947**
**AU-B2- 2014 250 616**
**US-A1- 2014 199 193**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### Technical Field

The present invention relates to signal filtering device and a medical system comprising such a device in accordance with the appended claims.

### Background Art

In an apparatus for extracorporeal blood processing, blood is taken out of a human subject, processed (e.g. treated) and then reintroduced into the subject by means of an extracorporeal blood flow circuit ("EC circuit") which is part of the apparatus. Generally, the blood is circulated through the EC circuit by a blood pump. The EC circuit may include an access device for blood withdrawal (e.g. an arterial needle or catheter) and an access device for blood reintroduction (e.g. a venous needle or catheter), which are inserted into a dedicated vascular access (e.g. fistula or graft) on the subject.

It is vital to minimize the risk for malfunction of such an apparatus, since malfunctions may lead to a potentially life-threatening condition of the subject. Serious conditions may e.g. arise if the EC circuit is disrupted downstream of the blood pump, e.g. by a Venous Needle Dislodgement (VND) event, in which the venous needle comes loose from the blood vessel access. Such a disruption may cause the subject to be drained of blood within minutes. WO97/10013, US2005/0010118, WO2009/156174, WO2010/149726 and US2010/0234786 all propose various techniques for detecting a VND event by identifying an absence of heart or breathing pulses in a pressure signal from a pressure sensor ("venous sensor") on the downstream side of the blood pump in the EC circuit.

Recently, it has also been shown to be possible to monitor and analyze the behavior of physiological pulse generators such as the heart or respiratory system, based on pressure recordings in the EC circuit. Various applications are found in WO2010/149726, WO2011/080189, WO2011/080190, WO2011/080191, WO2011/080194 and WO2014/147028. For example, these applications include monitoring a subject's heart pulse rate, blood pressure, heart rhythm, cardiac output, blood flow rate through the blood vessel access ("access flow"), arterial stiffness, as well as identifying signs of stenosis formation within the blood vessel access, predicting rapid symptomatic blood pressure decrease, detecting cardiac arrest, and detecting, tracking and predicting various breathing disorders. Furthermore, WO2011/080188 proposes a technique for identifying and signaling a reverse placement of the access devices in the vascular access by detecting and analyzing physiological signal components in a pressure signal recorded in the EC circuit.

All of these monitoring techniques presume that the physiological signal components can be reliably detected in the pressure signal. To enable monitoring, it may be necessary operate a filter on the pressure signal for removal or suppression of signal interferences, e.g. pressure pulses ("pump pulses") originating from the blood pump. Numerous filtering techniques have been proposed for suppressing the pump pulses, e.g. as described in WO97/10013, WO2009/156175, WO2013/000777, WO2016/206949, WO2014/009111 and WO2015/032948. While these and other filtering techniques are capable of suppressing the pump pulses, they are typically sensitive to disturbances in the pressure signal, in that the disturbances may cause significant and extended ringing artifacts in the filtered signal. The ringing artifacts may have a negative impact of the monitoring performance, and may even make monitoring impossible for an extended time period.

It should be noted that the problem of ringing artifacts is not limited to filtering techniques designed to suppress pump pulses, but may be equally relevant to filtering techniques designed to suppress other signal interferences in a pressure signal from a pressure sensor in the EC circuit. Further, the problem is not limited to extracorporeal blood processing but may also arise in relation to other types of medical apparatuses.

There is thus a general need for a filtering technique with an improved robustness to disturbances in a pressure signal that represents fluid pressure in a medical apparatus.

The prior art also comprises US2014/0199193, WO2014/035947, WO2013/000777, AU2014250616 and WO2012/170119.

### Summary

It is an objective of the invention to at least partly overcome one or more of limitations of the prior art, by providing a signal filtering device and a medical device comprising such a device in accordance with the appended claims.

### Brief Description of Drawings

FIG. 1 is a schematic diagram of a medical system comprising an extracorporeal blood processing apparatus and a filtering device.
FIG. 2 is a side view of a rotor of a peristaltic pump in the apparatus in FIG. 1.
FIG. 3 is a plot of a pressure signal generated by a pressure sensor in the medical system of FIG. 1.
FIGS 4A-4B are plots of exemplifying filter characteristics of a digital filter in the filtering device in FIG. 1.
FIG. 5A is an illustrative structure of a digital filter, and FIG. 5B exemplifies computation of a time-sequence of filtered data samples and state vectors for the digital filter in FIG. 5A.
FIG. 6 is a block diagram of a filtering arrangement within the filtering device in FIG. 1.
FIG. 7 is a flow chart of a filtering process executed by the filtering arrangement of FIG. 6.
FIGS 8A-8B illustrate first and second embodiments for filter reconfiguration in the filtering process of FIG. 7, and FIG. 8C exemplifies the content of a database for use in the second embodiment.
FIG. 9 illustrates example signals for a filtering process involving a filter reconfiguration according to the first embodiment in FIG. 8A.
FIGS 10A-10B are plots of filtered pressure signals generated with and without a filter reconfiguration.
FIG. 11 is a schematic diagram of a medical system comprising an infusion apparatus and a filtering device.

## Detailed Description of Example Embodiments

Embodiments of the present invention will now be described more fully hereinafter with reference to the accompanying drawings. Like numbers refer to like elements throughout.

Any terms expressed in the singular form herein are meant to also include the plural form and/or vice versa, unless explicitly stated otherwise. As used herein, "at least one" shall mean "one or more" and these phrases are intended to be interchangeable. Accordingly, the terms "a" and/or "an" shall mean "at least one" or "one or more," even though the phrase "one or more" or "at least one" is also used herein. As used herein, except where the context requires otherwise owing to express language or necessary implication, the word "comprise" or variations such as "comprises" or "comprising" is used in an inclusive sense, that is, to specify the presence of the stated features but not to preclude the presence or addition of further features in various embodiments of the invention.

Before describing embodiments of the invention detail, a few further definitions will be given.

As used herein, "medical apparatus" denotes any apparatus, device, machine or system that is configured for diagnostic and/or therapeutic purposes, such as diagnosis, prevention, monitoring, treatment or alleviation of a disease, injury or handicap.

As used herein, "extracorporeal blood processing apparatus" denotes any apparatus, device, machine or system that is configured to take blood from the circulatory system of a human or animal subject, apply a process to the blood and then reintroduce the processed blood into the circulatory system. The extracorporeal blood processing apparatus may be configured for any type of blood treatment, including dialysis therapies such as hemodialysis (HD), hemofiltration (HF) hemodiafiltration (HDF), and ultrafiltration (UF), as well as heart congestion failure treatment, plasmapheresis, apheresis, extracorporeal membrane oxygenation, assisted blood circulation, extracorporeal liver support/dialysis, respiratory dialysis, etc.

As used herein, "infusion apparatus" denotes any apparatus, device, machine or system that is configured or specifically adapted for introducing an infusion fluid into a human or animal subject, e.g. into the circulatory system of the subject, e.g. intravenously, or by subcutaneous, arterial or epidural infusion, or into a cavity of the subject, such as the abdomen. The infusion fluid may include any medical fluid or combination of medical fluids, such as resuscitation fluid, medication, nutrient, blood, peritoneal dialysis fluid, etc.

As used herein, "vector" is given its ordinary meaning and denotes a data structure comprising a collection of elements, each identified by an index or key.

As used herein, "digital filter" is given its ordinary meaning and denotes a system that performs mathematical operations on a sampled, discrete-time input signal to reduce or enhance certain features of the input signal. A digital filter is implemented by program instructions running on a processor that operates in conjunction with a digital storage memory to perform mathematical operations on data samples in the input signal. The digital filter may be implemented as an infinite impulse response (IIR) filter (also known as a recursive filter) or a finite impulse response filter (FIR), which are both well-known in the art.

As used herein, "processor" is given its ordinary meaning and denotes one or more electronic circuits that perform operations on data, e.g. in a digital storage medium or a data stream. The processor may, e.g., include one or more of a CPU ("Central Processing Unit"), a microprocessor, a DSP ("Digital Signal Processor"), a GPU ("Graphics Processing Unit), a combination of discrete analog and/or digital components, an ASIC ("Application-Specific Integrated Circuit"), an FPGA ("Field Programmable Gate Array"), etc.

As used herein, "digital storage memory" or "storage memory" is given its ordinary meaning and denotes any technology used to place, keep, and retrieve digital data. The digital storage memory may include volatile and/or non-volatile memory such as a hard disk, a removable magnetic disk, a removable optical disk, magnetic cassettes or other magnetic storage devices, flash memory, CD-ROM, digital versatile disks (DVD) or other optical storage, read only memory (ROM), random access memory (RAM), electrically erasable programmable read-only memory (EEPROM), quantum memory, etc.

As used herein, "computer-readable medium" is given its ordinary meaning and denotes any tangible (non-transitory) product or carrier that may be used for providing program instructions (software) to a processor, including a digital storage memory as exemplified above. The computer-readable medium may also be non-tangible such as a propagating carrier signal.

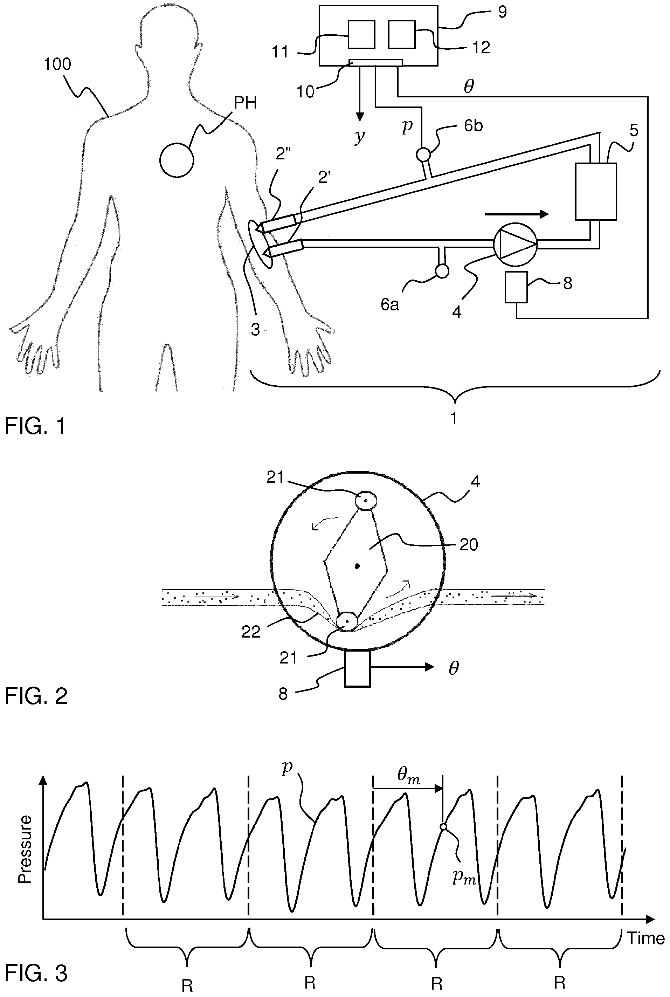

FIG. 1 illustrates a human subject or patient 100 which is connected to an extracorporeal fluid circuit (denoted "EC circuit" in the following) by way of access devices 2', 2" (e.g. needle, catheter) inserted into a dedicated vascular access 3 (also known as "blood vessel access") on the subject 100. As indicated in FIG. 1, the EC circuit is part of an apparatus 1 for blood processing, such as a dialysis machine. The EC circuit is configured to communicate blood to and from the cardiovascular system of the subject 100. In the illustrated example, a blood pump 4 is operable to draw blood from the vascular access 3 via an access device 2' for blood withdrawal and to pump the blood through a blood processing unit 5, e.g. a dialyzer, and back to the vascular access 3 via an access device 2" for blood return. Thus, when both access devices 2', 2" are connected to the vascular access 3, the EC circuit defines a blood path that starts and ends at the vascular access 3. The EC circuit may be seen to comprise a "venous side" which is the part of the blood path located downstream of the blood pump 4, and an "arterial side" which is the part of the blood path located upstream of the pump 4.

Pressure sensors 6a and 6b are arranged to detect fluid pressure in the apparatus 1. In the illustrated example, the sensors 6a, 6b are in direct or indirect hydraulic contact with the blood in the EC circuit and generate a respective time-varying pressure signal. The pressure sensors 6a, 6b are configured to generate the respective pressure signal to include a variety of frequency components. Thus, the pressure signals may be seen to include a slowly varying average pressure level (also known as "DC pressure" or "baseline pressure") and overlaid pressure variations on shorter time scales. The pressure variations may originate from one or more repetitive pulse generators. In the example of FIG. 1, such repetitive pulse generators may include the blood pump 4 and one or more physiological pulse generators PH in the subject 100, e.g. the heart or the breathing system. Although not shown in FIG. 1, the apparatus 1 may include further repetitive pulse generators, such as balancing chambers, further fluid pumps, fluid valves, etc. The pressure signals may also include non-repetitive pressure variations, e.g. caused by switching of fluid valves, the subject coughing, moving, etc.

The following description presumes that the dominant pressure variations in the respective pressure signal are repetitive and originate from the blood pump 4. Further, in the following examples, the blood pump 4 is presumed to be a rotary peristaltic pump of the type shown in FIG. 2. As is well-known in the art, a rotary peristaltic pump 4 is a positive displacement pump that comprises a rotor 20 with rollers 21 (also known as "shoes", "wipers", or "lobes") attached to the external circumference of the rotor 20. The blood pump 4 may have any number of rollers 21, e.g. two as shown in FIG. 2. The rollers 21 are arranged to engage, by rotation of the rotor 20, a tube segment 22 which is thereby pinched closed ("occluded") to force the fluid to be pumped to move through the tube segment 22.

In the examples of FIGS 1-2, a phase sensor 8 is associated with the blood pump 4 to generate a phase signal $\theta$ that indicates one or more angular positions of the rotor 20 during a revolution of the rotor 20. For example, the phase sensor 8 may generate a pulse in the phase signal $\theta$ for each of a number of predefined angular positions of the rotor. Such a pulse sensor is thus absolute in that it indicates well-defined positions. In one implementation, the phase sensor 8 comprises at least one Hall effect sensor which produces a pulse whenever passed by a magnet (not shown) attached to or integrated with the rotor 20. Such a phase sensor 8 indicates a single angular position for each Hall effect sensor. In another implementation, the phase sensor 8 comprises a rotary encoder which may be absolute or incremental (relative). The absolute rotary encoder indicates the current angular position of the rotor 20, whereas the incremental rotary encoder indicates the motion of the rotor 20. One example of an incremental rotary encoder is a tachometer. The tachometer produces a count of pulses during every rotation of the rotor 20 and restarts the count for each rotation. Since the number of pulses for each revolution of the rotor 20 is fixed and predefined, each count may be associated with a specific relative angular position of the rotor 20.

FIG. 3 illustrates a time-resolved pressure signal $p$ acquired from the pressure sensor 6b in FIG. 1. As seen, the pressure signal $p$ is dominated by pulsations originating from the blood pump 4, where each such pulsation ("pump pulse") corresponds to a respective roller 21 engaging the tube segment 22. Thus, the blood pump 4 may be seen to operate in a repeating sequence of pulse cycles R. In FIG. 3, each pulse cycle R corresponds to a revolution of the rotor 20 in FIG. 2 and thus contains two pump pulses (since the rotor 20 comprises two rollers 21). FIG. 3 also illustrates a data sample ("pressure sample") $p_m$ in the pressure signal $p$ and a phase value $\theta_m$ associated with the pressure sample $p_m$. It is understood that each angular position indicated by the phase signal $\theta$ corresponds to a respective location within the pulse cycle R.

Returning to FIG. 1, a filtering device 9 is connected to the apparatus 1 and configured to acquire the pressure signal $p$ from the pressure sensor 6b and operate a digital filter on the pressure signal $p$ to generate a corresponding filtered signal $y$. It should be understood that the filtering device 9 may alternatively or additionally operate the digital filter on a pressure signal from the pressure sensor 6a or another pressure sensor in the apparatus 1, e.g. a so-called system sensor that may be located between the pump 4 and the dialyzer 5, or a pressure sensor located in a system for providing a flow of dialysis fluid through the dialyzer 5. In the illustrated embodiment, the filtering device 9 comprises a signal interface 10, a processor 11 and a storage memory 12. Embodiments of the invention may e.g. be at least partly implemented by software instructions that are supplied on a computer-readable medium for execution by the processor 11 in conjunction with the storage memory 12. By the software instructions, the filtering device 9 is configured or programmed to implement embodiments of the invention. The signal interface 10 may be any hardware component or combination of components for input, and optionally output, of electrical or optical signals.

The filtering device 9 may be included as part of the apparatus 1, e.g. connected to or part of a main control unit for the apparatus 1. Alternatively, the filtering device 9 may be separate from the apparatus 1.

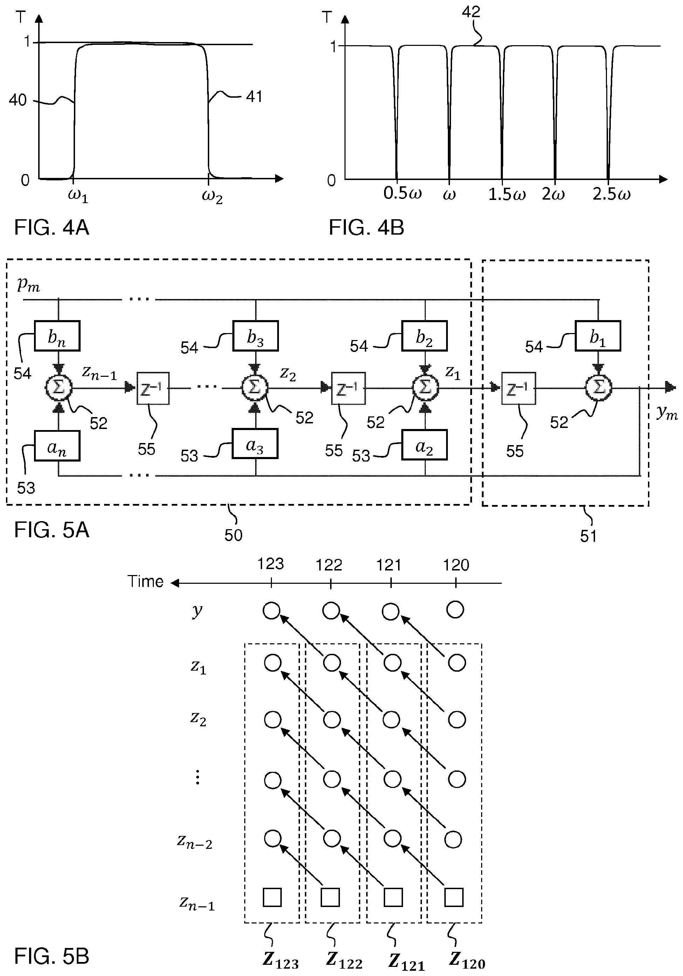

The digital filter in the filtering device 9 may be of any conceivable type, e.g. a high-pass filter, a low-pass filter, a bandpass filter, a notch filter, a comb filter, etc. As an example, FIG. 4A illustrates a transmission curve 40 for a high-pass (HP) filter with a cut-off frequency $\omega_1$ and a transmission curve 41 for a low-pass (LP) filter with a cut-off frequency $\omega_2$. In the context of FIG. 1, the HP filter may be operated on the pressure signal *p* to remove low-frequency noise, and optionally the baseline pressure, and the LP filter may be operated on the pressure signal *p* to suppress noise and other highfrequency disturbances. FIG. 4B illustrates a transmission curve 42 for a comb filter which is configured to block a base frequency $\omega$ and its harmonics, i.e. $0.5\omega$, $1.5\omega$, $2\omega$, etc. In the context of FIG. 1, the comb filter may be configured to match the base frequency $\omega$ to the frequency of pump pulses generated by the blood pump 4 and operate the comb filter on the pressure signal to essentially block all frequency components of the pump pulses and thereby generate a filtered signal y essentially without pump pulses.

It is realized that the digital filter in the filtering device 9 may be a combination of two or more filter types. Alternatively, the filtering device 9 may operate two or more digital filters of different types in sequence, such that one digital filter is operated on the output signal of another digital filter to generate the filtered signal *y*.

By their nature, digital filters are sensitive to disturbances in the incoming pressure signal *p*. What is considered a disturbance depends on the desired filtering properties of the specific digital filter, but typically a disturbance causes the filtered signal *y*, subsequent to the disturbance, to deviate significantly from the actual (true) pressure at the location of the pressure sensor that produces the pressure signal *p* that is being filtered. Such deviations in the filtered signal *y* are also denoted "ringing artifacts" or "transients" herein. For strong disturbances, the decay of the ringing artifacts may be slow.

In one example, a sudden change to the operating condition of the repetitive pulse generator may cause an unwanted and significant disturbance in the pressure signal *p*. In the example of FIG. 1, the blood pump 4 generates strong pump pulses in the pressure signal *p*. Thus, whenever the blood pump 4 is stopped or started, strong ringing is likely to occur in the filtered signal y. For example, the apparatus 1 may be configured to temporarily stop the blood pump 4 as part of a procedure to verify a potential fault or alarm condition. Likewise, a change in pumping rate of the blood pump 4 may cause ringing in the filtered signal, especially a step change.

In another example, any of the above-mentioned non-repetitive pressure variations may form a disturbance that causes unwanted ringing in the filtered signal y. It is also conceivable that repetitive pulse variations cause unwanted ringing, especially if the interval between the pulse variations is long. For example, a valve that is switched every 5 or 10 minutes may generate unwanted ringing.

The ringing artifacts in the filtered signal *y* may obscure other signal features, thereby making it difficult or even impossible to analyze the filtered signal *y* for an extended time period after the disturbance. It is not unlikely that an automated analysis of the filtered signal *y* produces erroneous results in presence of significant ringing.

In the context of FIG. 1 and assuming that the filtering aims at producing a filtered signal *y* without pump pulses, such automated analysis may aim at detecting and processing signal components of physiological origin in the filtered signal *y*, e.g. pressure pulsations originating from one or more physiological pulse generators PH in the subject 100, e.g. the heart or the breathing system. For example, as described in the Background section, the physiological signal components may be analyzed for extraction of various physiological parameters for the subject and/or for identifying various disorders or unwanted physiological conditions of the subject. Further, as also described in the Background section, the physiological signal components may be analyzed for detection of a VND event. Assuming that the filtering retains the pump pulses, the automated analysis may alternatively process the pump pulses for detection of malfunctions in the blood pump 4, stenosis formation within the vascular access 3, a VND event, etc. In a further alternative, the automated analysis may aim at detecting other signal features in the filtered signal, e.g. a change in baseline pressure corresponding to a VND event.

Embodiments of the invention as defined in the appended claims are based on the insight that it may be desirable to detect or predict the occurrence of a disturbance in the pressure signal and to selectively reconfigure the digital filter at a selected time point after the disturbance, e.g. when the disturbance has subsided in the pressure signal. embodiments are also based on the insight that the digital filter is advantageously reconfigured by updating the state vector of the digital filter, specifically by matching the state vector to the working point of the apparatus 1 at the selected time point. As used in this context, the working point of the apparatus 1 is specific to a time point and comprises current values of one or more parameters that affect or represent the pressure signal. As realized from FIG. 3, the phase of the blood pump 4 has a major impact on the pressure signal in the example of FIG. 1. Thus, in the following examples, the working point of the apparatus 1 is given by the phase of the blood pump 4.

As is well-known in the art, the filtering process in an IIR digital filter may be represented by the difference equation:

$$a_1 \cdot y(n) = b_1 \cdot p(n) + \cdots + b_{N+1} \cdot p(n-N) - a_2 \cdot y(n-1) - \cdots - a_{M+1} \cdot y(n-M) \quad (1)$$

where $a_1, ..., a_{M+1}$ and $b_1, ..., b_{N+1}$ are first and second filter coefficients, respectively, $p(n-1), ..., p(n-N)$ are the $N$ most recent preceding pressure samples in the pressure signal, and $y(n-1), ..., y(n-M)$ are the $M$ most recent preceding filtered samples. Eq. (1) is also known as the Direct-Form I (DF-I) implementation of a digital filter.

As is also well-known in the art, Eq. (1) may be re-written as a set of time-domain equations that relate the current filtered sample $y_m$ to the current pressure sample $p_m$ in the pressure signal via a set of state values for the digital filter:

$$\begin{aligned} y_m &= b_1 \cdot p_m + z_1^* \\ z_1 &= b_2 \cdot p_m + z_2^* - a_2 \cdot y_m \\ &\vdots \\ z_{n-2} &= b_{n-1} \cdot p_m + z_{n-1}^* - a_{n-1} \cdot y_m \\ z_{n-1} &= b_n \cdot p_m - a_n \cdot y_m \end{aligned} \quad (2)$$

where $z_1, ..., z_{n-1}$ are state values for the digital filter at the current time point, and $z_1^*, ..., z_{n-1}^*$ are state values for the digital filter at a preceding time point. It may be noted that the values of the filter coefficients may differ between Eq. (1) and Eq. (2). It may also be noted that Eq. (2) presumes an equal number of first and second filter coefficients, $N = M = n$.

Eq. (2) is also known as the Direct-Form II (DF-II) implementation of the digital filter and defines the operation of the digital filter in terms of a state vector $\boldsymbol{Z}$ containing the state values $z_1, ..., z_{n-1}$, a first coefficient vector $\boldsymbol{A}$ containing the first filter coefficients $a_1, ..., a_n$, and a second coefficient vector $\boldsymbol{B}$ containing the second filter coefficients $b_1, ..., b_n$. At least the state vector $\boldsymbol{Z}$ is re-calculated at predefined time steps that correspond to a predefined number of pressure samples in the pressure signal, and preferably for each pressure sample.

As is known in the art, the Direct-Form II (DF-II) implementation of the digital filter may be represented by a system diagram as shown in FIG. 5A. In the system diagram, the digital filter is composed of summation units 52, multiplication units 53, 54 and delay units 55. Specifically, the digital filter comprises a state engine 50 and a filter output generator 51. The state engine 50 comprises summation units 52 arranged in sequence, and a respective delay unit 55 between each pair of summation units 52 in the sequence. The respective delay unit 55 is configured to delay an output of the respective summation unit 52 by one time step before being received as input by a subsequent summation unit 52 in the sequence. The respective summation unit 52 receives, as input, the current filtered sample $y_m$ modified, in a respective multiplication unit 53, by a respective first filter coefficient $a_2, ..., a_n$ and the current pressure sample $p_m$ modified, in a respective multiplication unit 54, by a respective second filter coefficient $b_2, ..., b_n$. The respective summation unit 52 generates the output as a sum of the inputs. The output of the respective summation unit 52 in the state engine 50 is a state value $z_1, ..., z_{n-1}$ of the digital filter. The filter output generator 51 comprises a summation unit 52 that receives the current pressure sample $p_m$ modified, in a multiplication unit 54, by the second filter coefficient $b_1$ and, via a delay unit 55, a first state value $z_1$ produced by the state engine 50. The output of the summation unit 52 is the current filtered sample $y_m$.

It should be understood that the system diagram of FIG. 5A is a primarily a model for visualizing the digital filter. In practice, the program instructions that implement and control the digital filter need not define the structure in the system diagram. Instead, the program instructions may implement the computations in accordance with Eq. (2). The computations in accordance with Eq. (2) are visualized in FIG. 5B at four consecutive time points 120, 121, 122 and 123. The state values $z_1, ..., z_{n-1}$ of the digital filter at each time point are represented by a state vector, designated $\boldsymbol{Z_{120}}$, $Z_{\boldsymbol{121}}$, $Z_{\boldsymbol{122}}$ and $Z_{\boldsymbol{123}}$ in FIG. 5B. The arrows indicate how the filtered sample in the filtered signal y depends on the state values and how the previous state values are applied for generating a current state vector at the respective time step. As seen, at each time point, the current state vector is computed as a function of the preceding state vector, and the filtered data sample is computed as a function of the preceding state vector, in this example the state value $z_1$.

In the following, for simplicity of notation and in correspondence with Eq. (2), the current state vector is designated $\boldsymbol{Z_m}$ and comprises state values $z_1, ..., z_{n-1}$, and the preceding state vector is designated $\boldsymbol{Z^*}$ and comprises state values $z_1^*, ..., z_{n-1}^*$. In accordance with Eq. (2) and the computational flow in FIG. 5B, the digital filter may be configured and operated to repeatedly, at each current time, compute a current filtered sample $y_m$ as a function of the current pressure sample $p_m$, the preceding state vector ***Z**** (e.g. state value $z_1^*$ ), and the second coefficient vector ***B*** (e.g. coefficient $b_1$). The digital filter may also be configured and operated to repeatedly, at each current time, compute a current state vector $\boldsymbol{Z_m}$ as a function of the preceding state vector ***Z*****,** the first and second coefficient vectors ***A, B,*** the current pressure sample $p_m$ and the current filtered sample $y_m$.

Although the foregoing discussion has been given in relation to an IIR digital filter, it is equally applicable to a FIR digital filter by setting the first filter coefficients $a_1, \ldots, a_{n-1}$ to zero (0).

The number of states, n, may depend on filter type and the required performance of the digital filter. For example, the HP or LP filters in FIG. 4A may be configured with a small number of states, e.g. $n = 4 - 10$, whereas the comb filter in FIG. 4B may need to be configured with a larger number of states, e.g. $n = 30 - 60$. As understood from Eq. (2) and Fig. 5B, each instance of the state vector ***Z*** is affected by a number of preceding pressure samples, and this number increases in proportion to the number of states. This means that the sensitivity of the digital filter to disturbances in the pressure signal may be seen to increase with increasing number of states, since the disturbance will have an impact on the state vector ***Z*****,** and thus on the filtered signal y, for a longer time.

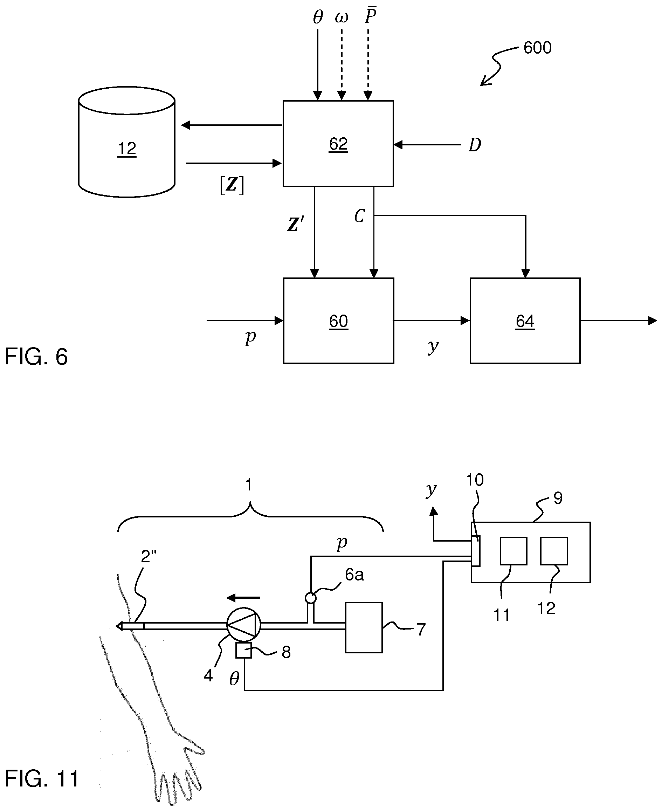

FIG. 6 is a block diagram of a signal filtering arrangement 600 within the filtering device 9 in FIG. 1. The arrangement 600 comprises a digital filter 60, which is configured to receive the pressure signal *p*. Although not shown in FIG 6, the pressure signal *p* may be pre-processed by a dedicated unit in the signal filtering arrangement, e.g., by AD conversion, signal amplification, etc. The digital filter 60 is configured in accordance with the above-described Direct-Form II (DF-II) implementation, so as to generate a filtered signal y by repeatedly computing the state vector ***Z*** and operating the state vector ***Z*** on the pressure signal *p*. The digital filter 60 is further designed to allow a filter controller 62 to selectively reconfigure the filter 60, by modifying the state vector ***Z*** so as to reduce the influence of the disturbance on the filtered signal *y*. The filter controller 62 is designed to reconfigure the filter 60 based on a trigger signal *D*, which is indicative of an actual or expected disturbance in the pressure signal *p*. In one example, the trigger signal *D* may be or comprise a control signal for a functional component of the apparatus 1, such as a valve, the blood pump 4, etc. In another example, the trigger signal *D* is or comprises a software command for a mode shift as generated by the main controller of the apparatus 1, e.g. to switch the apparatus 1 to or from a bypass mode, or to or from a mode that stops the blood pump 4. In yet another example, the trigger signal *D* may result from an analysis of the pressure signal *p* or another sensor signal in the apparatus 1.

In one embodiment, the filter controller 62 is configured to selectively modify the preceding state vector ***Z**** of the digital filter 60 at a selected time point when the disturbance has or is expected to have disappeared or significantly subsided in the pressure signal *p*. The filter controller 62 may detect or predict the existence of the disturbance based on the trigger signal *D*. In the illustrated embodiment, the filter controller 62 is configured to reconfigure the digital filter 60 by replacing the preceding state vector ***Z**** of the digital filter 60 at the selected time point by a dedicated reconfiguration state vector ***Z'*** ("reconfiguration vector"), which differs from the preceding state vector ***Z**** and which is preferably unaffected by the disturbance. The filter controller 62 is configured to obtain the reconfiguration state vector ***Z'*** as a function of one of more state vectors **[*Z*]** retrieved from the memory 12. Specifically, the filter controller 62 is configured to obtain a reconfiguration vector ***Z'*** that is matched to the working point of the apparatus 1 at the selected time point. In the illustrated example, and as discussed above, the working point is at least given by the phase of the blood pump 4, as indicated by the phase signal $\theta$. As will be described further below and is indicated in FIG. 6, the working point may be further given by the pumping rate of the blood pump 4, as indicated by a signal $\omega$, and/or an average fluid pressure in the apparatus 1, as indicated by a signal $\overline{P}$, and/or an amplitude of pressure variations in the pressure signal *p*.

As indicated in FIG. 6, the filter controller 62 may be further configured to generate and provide a control signal *C* that indicates presence of the disturbance in the pressure signal *p*. The filter controller 62 may generate the control signal *C* based on the trigger signal *D*.

In one embodiment, as shown, the control signal *C* may be provided by the filter controller 62 to the filter 60 so as to thereby cause the filter 60 to stop its operation during the disturbance. This means that the filter 60 does not output any filtered samples during the disturbance. For example, the control signal *C* may indicate a first time point when the filter 60 is to be stopped, and optionally a second time point when the filter is to be restarted and reconfigured by use of the reconfiguration vector ***Z'*****.** Alternatively, the second time point is given by the time point when the filter controller 62 enters the reconfiguration vector ***Z'*** into the filter 60.

In one embodiment, as shown, the control signal *C* is provided to a post-processing unit 64 in the signal filtering arrangement. The post-processing unit 64 may be configured to modify the filtered signal *y* based on the control signal *C*. If the filter 60 is stopped during the disturbance, the post-processing unit 64 may be controlled to add fictitious signal values (e.g. 0) to the filtered signal *y* during the stop. If the filter 60 is not stopped during the disturbance, the post-processing unit 64 may be configured to change the data samples in the filtered signal *y* during the disturbance, e.g. to a predefined value (e.g. 0), so as to prevent filtered values that are potentially corrupt from being subsequently used in an automated analysis of the filtered signal *y*.

It should be emphasized that the post-processing unit 64 may be omitted. It is also conceivable that the control signal *C* (if generated) is provided as input to the automated analysis, so as to indicate potentially corrupt data samples in the filtered signal *y*.

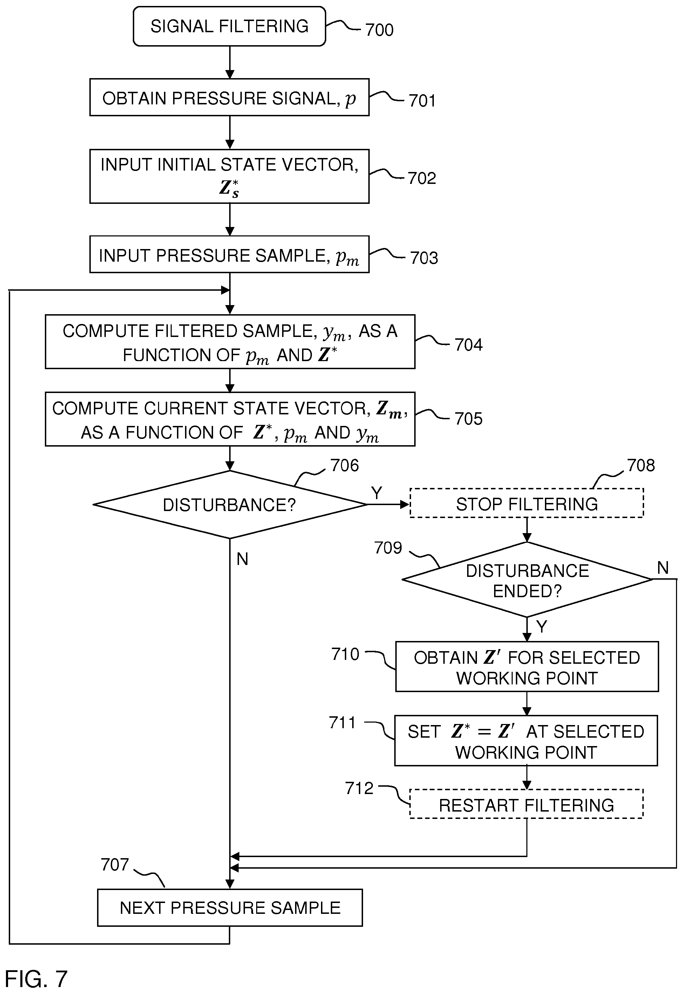

FIG. 7 is a flow chart of an embodiment of a signal filtering method 700, not part of the claimed subject-matter, which may be executed by the filtering device 9 in FIG. 1 and the arrangement 600 in FIG. 6. The method 700 is typically executed in on-line, i.e. while the pressure samples are generated in the pressure signal *p*. However, it is also conceivable to execute the method 700 off-line, i.e. for filtering a previously recorded pressure signal *p*. The method comprises a step 701 of obtaining a pressure signal *p* comprising a time-sequence of pressure samples. It is realized that step 701 may be executed continuously or intermittently during execution of the method 700. The method 700 repeatedly executes a sequence of steps 704-707, and may intermittently branch into executing one or more of steps 708-712. Thereby, the method 700 processes the pressure samples in the pressure signal *p* one by one for generation of a corresponding filtered sample, except if the digital filter 6 is intermittently stopped (step 708 below). Each execution of the method from step 704 through step 707 is denoted an "iteration" of the method in the following. Steps 702-707 define the filtering operation that is executed by the digital filter 60 in FIG. 6, and steps 708-712 result in a reconfiguration of the digital filter 60 and may be implemented by the filter controller 62 in FIG. 6.

In the specific example of FIG. 7, step 702 retrieves an initial state vector $\boldsymbol{Z_s^*}$ to be used as $\boldsymbol{Z^*}$ at start-up of the method, and step 703 acquires a current pressure sample $p_m$ from the pressure signal p. The initial state vector $\boldsymbol{Z_s^*}$ may be hardcoded or retrieved from memory 12. Step 704 computes the current filtered sample $y_m$ in accordance with Eq. (2), i.e. as a function of $p_m$, $\boldsymbol{Z^*}$ (e.g. $z_1^*$) and $\boldsymbol{B}$ (e.g. $b_1$). Step 705 computes a current state vector $\boldsymbol{Z_m}$ in accordance with Eq. (2), i.e. as a function of $\boldsymbol{Z^*}$, $\boldsymbol{A}$, $\boldsymbol{B}$, $p_m$ and $y_m$. Step 706 detects or predicts if a disturbance has occurred in the pressure signal *p*, based on the trigger signal *D*. In one example, step 706 may predict a disturbance whenever the trigger signal *D* indicates an operational change of the apparatus 1 that is known to cause a disturbance in the pressure signal, or a predefined time period after such an operational change. In another example, the trigger signal *D* may be the pressure signal *p*, or a signal derived therefrom, and step 706 may process the trigger signal D for detection of an actual disturbance.

If no disturbance has occurred according to step 706, the method proceeds to step 707, which acquires the next pressure sample from the pressure signal p, whereupon the method returns to step 704. In step 704, the current pressure sample $p_m$ is given by the pressure sample acquired in preceding step 707, and the preceding state vector $\boldsymbol{Z^*}$ is given by the current state vector $\boldsymbol{Z_m}$ computed in preceding step 705.

If step 706 determines that a disturbance has occurred, the method proceeds to step 709, optionally via step 708 (below). Step 709 then determines if the disturbance detected by step 706 is on-going in the pressure signal p or if the disturbance has disappeared or at least significantly subsided. In one example, step 709 may be deterministic and consider a disturbance to be on-going for predefined delay period (e.g. given as a number of pressure samples) from the time point when step 706 indicates a disturbance. Step 709 may apply different delay periods for different types of disturbances. The delay periods may be predefined and obtained by testing for the individual apparatus 1 or a type of apparatuses 1. In another example, step 709 may verify disappearance of the disturbance by analysis of the pressure signal p, or a signal derived therefrom.

If step 709 indicates that the disturbance is on-going, the method proceeds to step 707, which acquires the next pressure sample from the pressure signal *p*, whereupon the method returns to step 704. Thereby, as long as the disturbance is on-going, the method repeatedly performs iterations to produce one current filtered sample $y_m$ for each pressure sample $p_m$.

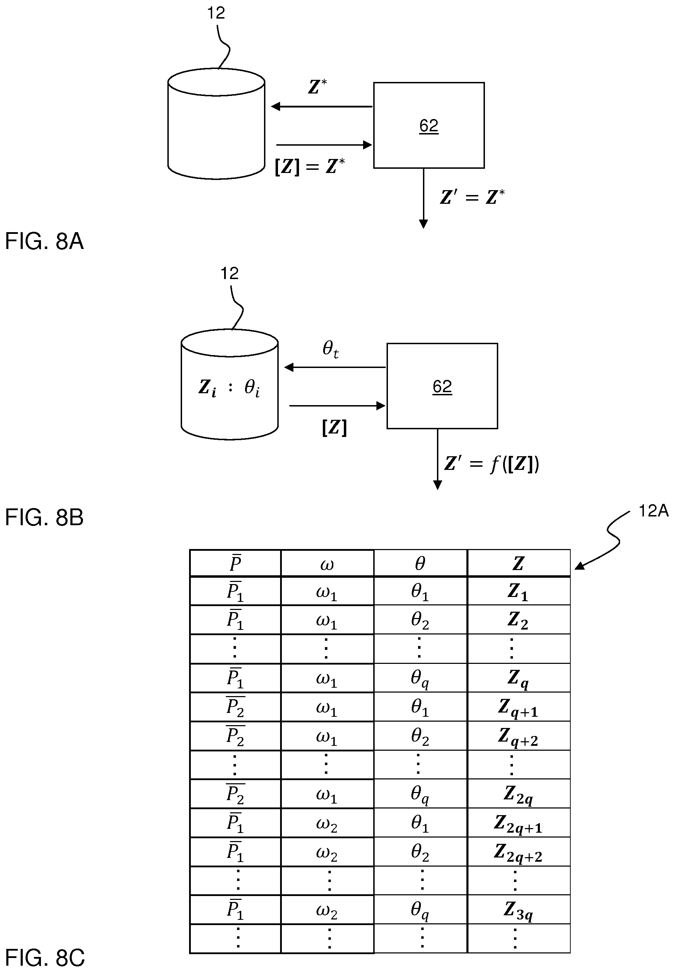

If step 709 indicates that the disturbance has ended, the method proceeds to step 710, which initiates a process of obtaining a reconfiguration vector ***Z'*** for a selected working point, e.g. a selected phase value of the blood pump 4. As described with reference to FIG. 6, step 710 may involve deriving the reconfiguration vector ***Z'*** as a function of a set of state vectors **[*Z*],** which are retrieved from a database stored in memory 12. Embodiments of step 710 are described in more detail below with reference to FIGS 8A-8C. It is important to note that the process of obtaining ***Z'*** according to step 710 may extend over more than one iteration of the method. It should also be understood that step 710 may be executed in advance of step 709, so that the process of obtaining ***Z'*** is initiated and possibly completed before step 709 indicates that the disturbance has disappeared.

Following completion of step 710, the method executes a step 711 which reconfigures the filter 60 by setting $\boldsymbol{Z^*}$ equal to ***Z'*.** Thereby, ***Z'*** will be used as the preceding state vector $\boldsymbol{Z^*}$ in the next execution of step 704. It should be noted that step 711 is executed only for the selected working point, e.g. as indicated by the phase signal $\theta$. Thus, the method may perform any number of iterations from the time point when the disturbance has disappeared, according to step 709, to the execution of step 711.

As indicated by a dashed box in FIG. 7, the method 700 may include an optional step 708 of stopping (disabling) the filtering performed by steps 704-705 during the disturbance in the pressure signal. In such a variant, step 708 may cause in the method 700 to refrain from executing steps 704-705 for a number of consecutive pressure samples in the pressure signal. While the filtering has been stopped by step 708, the method may operate in the same way as described above with respect to steps 709-711. Thus, the method 700 waits for the disturbance to disappear (step 709), initiates a process of obtaining a reconfiguration vector ***Z'*** for a selected working point (step 710), and sets ***Z**** equal to ***Z'*** at the selected working point (711). As noted above, step 710 may be executed in advance of step 709. Step 711 is followed by a step 712, which restarts the filtering by allowing the method to proceed to step 704 via step 707. Step 712 is preferably executed in the same iteration as step 711.

FIG. 8A illustrates a first embodiment for obtaining the reconfiguration vector ***Z'*** according to step 710. In FIG. 8A, the first embodiment is executed by the filter controller 62 in relation to the memory 12. The first embodiment presumes that the method 700 includes an additional step of storing the preceding state vector ***Z**** in memory 12 at a first time point before the disturbance significantly affects the preceding state vector ***Z*****,** e.g. before step 706 indicates the occurrence of a disturbance, or shortly thereafter, e.g. in the same iteration. The additional step also sets the selected working point (as used by step 711) to the current working point of the apparatus 1 at the first time point. The current working point may be given by a current phase value obtained from the phase signal $\theta$. In the first embodiment, the set of state vectors **[*Z*]** retrieved by step 710 may consist of the stored ***Z*****,** and thus ***Z'*** is equal to ***Z*****.** Step 711 waits to reconfigure the filter by ***Z'*** until a second time point when the apparatus 1 is in the selected working point, as indicated by the phase signal $\theta$. It should be noted that the selected working point need not be determined when the phase signal $\theta$ indicates a single phase value per revolution of the rotor 20 (FIG. 2). In this specific case, step 710 may be defined to store ***Z**** in an iteration when the phase signal indicates this single phase value, and the stored ***Z**** may thereby be implicitly associated with this single phase value.

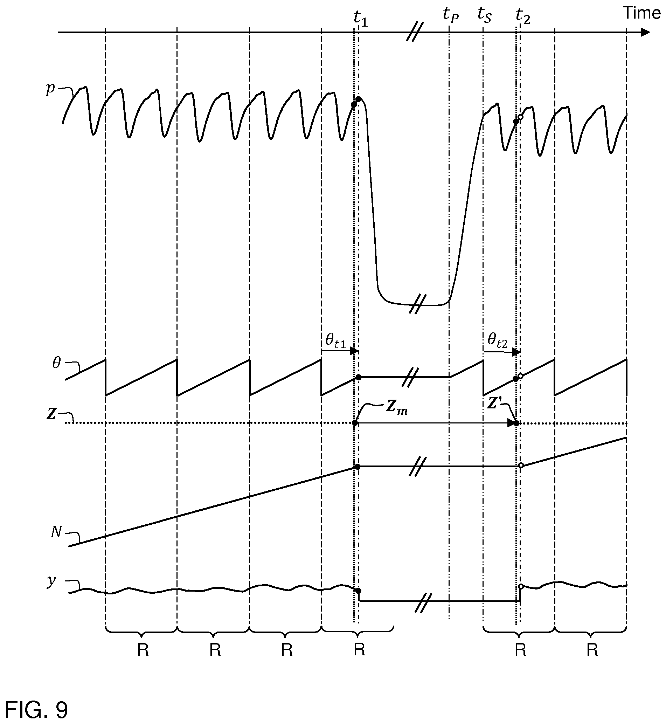

The first embodiment is exemplified in FIG. 9, which is a plot of signals used and generated during execution of the method 700 in FIG. 7. The top-most signal in FIG. 9 illustrates a pressure signal *p* which is input for filtering by the method 700. The pressure signal *p* is dominated by pump pulses originating from the blood pump 4 that operates in pulse cycles R (indicated by vertical dashed lines). At a first time point $t_1$ (indicated by a vertical dash-dot line), the blood pump 4 is stopped. As seen, this causes the pump pulses to disappear and the baseline pressure to drop. At a subsequent time point $t_P$ (indicated by a vertical dash-dot-dot line), the blood pump 4 is re-started, causing the baseline pressure to increase and pump pulses to emerge in the pressure signal *p*. It should be noted that FIG. 9 is a simplified example and that in practice it may take several pulse cycles R until the baseline pressure is stable after a restart of the blood pump 4. FIG. 9 also illustrates the phase signal $\theta$, which in this example increases monotonically within the respective pulse cycle R and is reset at the onset of each pulse cycle R. FIG. 9 also includes a dotted horizontal line which is intended to represent, by individual dots, the current state vectors that are generated by step 705 during execution of the method. In the illustrated example, step 706 detects a disturbance at a first time point $t_1$, i.e. when the pump 4 is stopped. In accordance with the first embodiment of FIG. 8A, the method stores ***Z**** for the current working point, given by the phase value $\theta_{t1}$. In FIG. 9, ***Z**** at the first time point $t_1$ is represented by $\boldsymbol{Z_m}$ computed for the preceding iteration (indicated by a vertical dotted line). The current phase value $\theta_{t1}$ is the selected working point to be used by step 711. At time point $t_S$ (indicated by a vertical dash-dot-dot line), step 709 detects that the disturbance has disappeared and that the baseline pressure is stabilized. As indicated by an arrow in FIG. 9, step 710 obtains the stored ***Z**** for use as reconfiguration vector ***Z'*.** Step 711 determines a second time point $t_2$ (indicated by a vertical dash-dot line), at which the current working point, given by the phase value $\theta_{t2}$, is equal to the selected working point, $\theta_{t1}$, and reconfigures the filter by ***Z'*** at the second time point $t_2$, which causes steps 704 and 705 to use ***Z'*** as ***Z**** at the second time point $t_2$.

In the example in FIG. 9, step 708 stops the digital filter 60 at the first time point $t_1$ and step 712 re-starts the digital filter 60 at the second time point $t_2$. This is seen from a count signal *N* which is an incrementing count of the number of pressure samples that have been processed by the digital filter (via steps 704-705). Between the first and second time points $t_1$, $t_2$, the count signal *N* is constant and thus no pressure samples are filtered. This is also seen in the filtered signal *y*, which is set to a fixed value between the first and second time points $t_1$, $t_2$. In this example, the fixed values are set by a process in the post-processing unit 64.

FIG. 8B illustrates a second embodiment for obtaining the reconfiguration vector ***Z'*** according to step 710, from a database that comprises state vectors associated with phase values ($\boldsymbol{Z_i}$ : $\theta_i$). In the second embodiment, step 710 determines the selected working point, based on any conceivable criterion, and queries the database in the memory 12 to retrieve a set of state vectors **[*Z*]** associated with the selected working point. In the example shown in FIG. 8B, the selected working point is designated by a selected phase value $\theta_t$. As indicated in FIG. 8B, step 710 obtains ***Z'*** as a function of the set of state vectors [***Z***], i.e. ***Z'*** = *f*([***Z***]). In one example, the set of state vectors **[*Z*]** consists of a single state vector, and step 710 may set ***Z'*** equal to this single state vector. However, it is conceivable that the set of state vectors [***Z***] comprises more than one state vector. For example, step 710 may obtain ***Z'*** by computing an element-wise mean (optionally weighted) or median of the state vectors associated with the selected phase value $\theta_t$. Step 711 may then reconfigure the filter in the same way as described for FIG. 8A.

FIG. 8C shows an example of a database 12A that may be stored in the memory and queried in the second embodiment of FIG. 8B. As shown, the database 12A contains a set of different phase values $\theta_1,..., \theta_q$, each associated with a respective state vector ($q \geq 1$). In the illustrated example, the content of the database 12A allows steps 710-711 to define the selected working point by a triplet of parameter values, namely the phase $\theta$ of the blood pump 4, an average fluid

pressure $\overline{P}$ in the apparatus 1 and the pumping rate $\omega$ of the blood pump 4. For example, a query containing ($\theta_2$, $\omega_1$, $\overline{P}_2$) will return state vector $\boldsymbol{Z_{q+2}}$. The average fluid pressure $\overline{P}$ may be equal to the above-mentioned baseline pressure and may be obtained from the pressure signal, e.g. by computing an average of the pressure samples within a sliding window with a length equal to one or more pulse cycles R. Alternatively, the average fluid pressure $\overline{P}$ may be computed from another pressure signal, e.g. acquired from sensor 6a in FIG. 1. The pumping rate $\omega$ of the blood pump 4 may, e.g., be computed based on the phase signal $\theta$, obtained from a dedicated sensor associated with the blood pump 4, obtained from a control signal for the blood pump, or obtained by analysis of the pump pulses in the pressure signal p. In a further variant, not shown, the selected working point may be at least partly given by the amplitude of the pressure variations in the pressure signal, e.g. computed as the difference between minimum and maximum pressure within one or more pulse cycles R.

It should be understood that FIG. 8C is merely given as an example. Thus, the working point may be defined by any suitable parameter(s) in combination with or instead of the phase. Further, as noted above, each working point may be associated with more than one state vector. The database 12A may be implemented by any conceivable data structure, such as one or more tables, lists, arrays, associative arrays, graphs, trees, etc.

The content of the database 12A may be fixed and predefined. For example, the content of the database 12A may be predefined for a specific type of apparatus 1 or for each individual apparatus 1.

Alternatively, the database 12A may be based on a predefined database 12A that is updated while the apparatus 1 is operated. In one example, the method 700 may include an initial step that adjusts the state vectors in the database 12A as a function of one or more characteristics of the pressure signal, e.g. the DC level, while the apparatus 1 is operated. In another example, the method 700 may include a step that intermittently adjusts the state vectors in the database 12A based on the current state vectors that are computed by step 705 for the respective working point. In yet another example, the method 700 may include a step that intermittently adds a state vector to the database, or replaces an existing state vector, based on the current state vector that is computed by step 705 for a respective working point.

In a further alternative, the database 12A is generated anew each time the apparatus 1 is started. In one example, the method 700 comprises a step that intermittently stores, in the database 12A, state vectors computed by step 705 in association with appropriate working points.

As mentioned above in relation to FIG. 8B, it is conceivable that the database 12A stores more than one state vector for a working point. In one example, step 705 in FIG. 7 may be further configured to update the database 12A, for every k:th iteration of the method (k = 1 or larger), by the preceding state vector in association with the current working point (e.g. given by the phase signal $\theta$), e.g. according to a FIFO (First-In First-Out) principle, such that the database 12A always contains a predefined number $l$ ($l$ > 1) of recently computed state vectors for one or more working points. It is currently believed that the accuracy of the reconfiguration vector $\boldsymbol{Z'}$ may be improved if $\boldsymbol{Z'}$ is derived as a function of such recently computed state vectors in step 710.

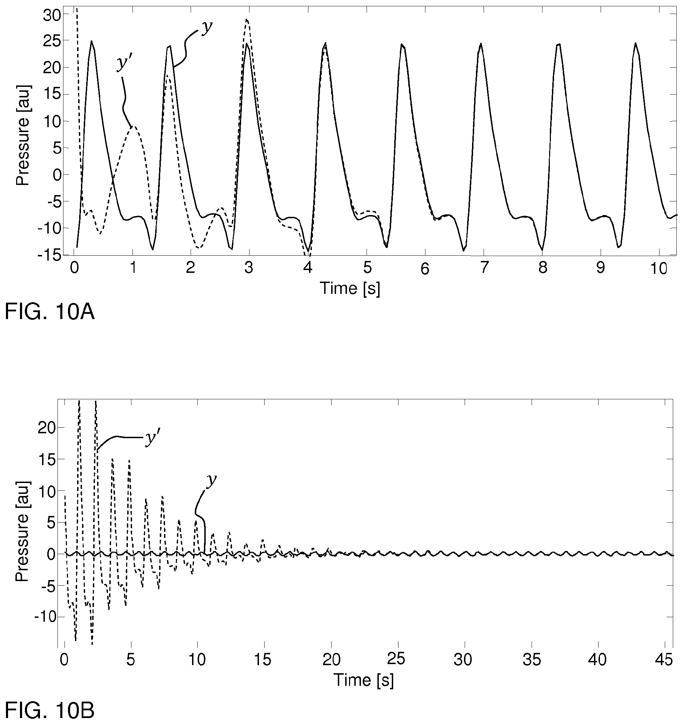

Embodiments of the invention provide the technical advantage of reducing the magnitude and/or duration of ringing artifacts in the filtered signal y, irrespective of the nature of the disturbance. This technical effect is exemplified in FIGS 10A-10B, which illustrate signals generated after a stop-restart operation of the blood pump 4: a filtered signal y generated by reconfiguration of the digital filter in accordance with steps 709-712 in FIG. 7, and a filtered signal *y*' generated without such reconfiguration. The reconfiguration of the digital filter is executed at time 0 in FIGS 10A-10B.

FIG. 10A is obtained for a HP filter (cf. FIG. 4A) which operates on the pressure signal *p* in FIG. 1 and is configured to pass frequencies of the pump pulses that are generated by the blood pump 4. As seen, filtered signal *y* reaches steady-state much faster than filtered signal *y*'.

FIG. 10B is obtained for a comb filter (cf. FIG. 4B) which operates on the pressure signal *p* in FIG. 1 and is configured to essentially eliminate the frequencies of the pump pulses that are generated by the blood pump 4. As seen, there is significant ringing in filtered signal y' caused by the stop-restart operation, whereas filtered signal y is essentially free of ringing after the reconfiguration. In the example of FIG. 10B, the filtered signals *y, y'* include weak pulsations that originate from the heart PH in the subject 100 (FIG. 1). As understood from FIG. 10B, these heart pulses are detectable in filtered signal y almost instantly after the reconfiguration, whereas the ringing in filtered signal y' hides or distorts the heart pulses for about 20 seconds.

Embodiments of the invention are generally applicable to medical apparatuses that comprise at least one pressure sensor for measuring fluid pressure. As a further example of such a medical apparatus, FIG. 11 shows an infusion apparatus 1 which comprises a fluid line that extends from a fluid source 7 to an access device 2" for insertion into a patient. An infusion pump 4 is arranged in the fluid line to pump an infusion fluid from the source 7 through the fluid line into the patient via the access device 2". A pressure sensor 6a is arranged to generate a pressure signal *p* representative of fluid pressure in the fluid line. A phase sensor 8 is arranged to generate a phase signal $\theta$ representative of the phase of the infusion pump 4. A filtering device 9 is connected to receive the pressure signal *p* and the phase signal $\theta$ and to output a filtered signal *y*. The skilled person realizes that foregoing description of embodiments in respect of the filtering device 9 in FIG. 1 and its operation is equally applicable to the filtering device 9 in FIG. 11.

While the invention has been described in connection with what is presently considered to be the most practical and preferred embodiments, it is to be understood that the invention is not to be limited to the disclosed embodiments, but on the contrary, is intended to cover various modifications included within the scope of the appended claims.

In the foregoing examples, the reconfiguration of the digital filter involves entering the reconfiguration vector ***Z'*** into the digital filter exactly at the selected working point (by step 711 in FIG. 7). However, in all embodiments disclosed herein, depending on the required suppression of ringing artifacts in the filtered signal y, it may be possible to deviate slightly from the selected working point, if desired or required for any reason. It is currently believed that adequate suppression of ringing artifacts may be achieved, at least in some embodiments, when the actual working point (phase value) at step 711 deviates from the selected working point (phase value) by less than $\pm5°$, preferably less than $\pm2°$, and most preferably less than $\pm1°$.

Depending on the configuration of the apparatus 1, it is conceivable that the working point of the apparatus 1 is given by the phase of a repetitive pulse generator other than the pump 4 in FIGS 1 and 11, provided that the filtering device 9 is able to deduce the current phase of this repetitive pulse generator from a phase signal or otherwise. In one example, the working point includes the phase of a physiological pulse generator PH in the subject 100 (FIG. 1), e.g. the heart, where the phase of the physiological pulse generator PH may be given by a dedicated sensor attached to the subject 100. It is further conceivable that the working point comprises the phases of two or more repetitive pulse generators, e.g. the pump 4 and an additional pump, or the pump 4 and the physiological pulse generator PH. This would mean that the database 12A (FIG. 8C) is expanded to associate each state vector with a combination of phase values, one for each pulse generator.

It is conceivable that the phase of the pulse generator is derived by processing more than one phase signal, e.g. by combining phase data from an incremental pulse sensor and phase data from an absolute pulse sensor. Such a combination may serve to increase the time resolution of the phase values. Further, the phase need not be derived from a phase signal of a pulse sensor 8. In an alternative, the phase may be derived from the pressure signal *p* to be filtered, or a pressure signal acquired from another pressure sensor (cf. 6a in FIG. 1), by processing the pressure signal for identification of one or more signal features that represent the phase of the pulse generator. For example, one or more phase values of the pump 4 during a pulse cycle R may be given by the time point(s) of one or more signal peaks in the pressure signal, a maximum or minimum slope in the pressure signal, a maximum correlation between the pressure signal and a reference profile for a pump pulse, etc. In a further alternative, the phase may be derived by combining time point(s) of signal features in the pressure signal with phase data from a phase sensor (absolute or incremental). In yet another alternative, the phase may be derived by combining phase data from an absolute phase sensor and a control signal that sets the speed of the pulse generator (e.g. pump 4). For example, a current phase value may be obtained by multiplying the current speed of the pulse generator by the time elapsed since the absolute phase sensor indicated an absolute phase value.

It may also be noted that the above-mentioned first and second filter coefficients may be either fixed or adapted during the filtering operation. If the digital filter is configured to adapt the filter coefficients, it is conceivable that the method 700 in FIG. 7 comprises an additional step that stores the current values of the filter coefficients in memory 12 when the disturbance is detected by step 706, and that the stored values are retrieved from memory 12 and applied when step 711 eventually reconfigures the filtering operation.

Further, the blood pump 4 need not be a rotary peristaltic pump as shown in FIG. 2, but could be of any conceivable type, including a linear peristaltic pump, a diaphragm pump, a piston pump, a screw pump, or a centrifugal pump. For volumetric pumps, the above-mentioned phase generally corresponds to a stroke position of the pump.

Still further, the pressure sensor may be of any type, e.g. operating by resistive, capacitive, inductive, magnetic, acoustic or optical sensing, and using one or more diaphragms, bellows, Bourdon tubes, piezo-electrical components, semiconductor components, strain gauges, resonant wires, accelerometers, etc. For example, the pressure sensor may be implemented as a conventional pressure sensor, a bioimpedance sensor, a photoplethysmography (PPG) sensor, etc.

## Claims

**1.** A signal filtering device, comprising a processor (11) and a data storage memory (12) storing computer instructions which, when executed by the processor (11), cause the processor (11) to:

obtain a pressure signal (p) comprising a time-sequence of data samples representing fluid pressure in a medical apparatus (1) for extracorporeal blood processing, in which blood is taken out of a human or animal subject, processed and then reintroduced into the human or animal subject by means of an extracorporeal blood flow circuit (1), said extracorporeal blood flow circuit (1) comprising a blood pump (4) for circulating the blood through the extracorporeal blood flow circuit (1), an access device (2') for blood withdrawal and an access device (2") for blood reintroduction, said access device (2') for blood withdrawal and said access device (2") for blood reintroduction being inserted into a dedicated vascular access of the human or animal subject;

operate a digital filter (60) on the pressure signal (*p*) to produce a filtered pressure signal (*y*) in which pressure pulses originating from the blood pump (4) are removed or suppressed, wherein the digital filter (60) is configured to, at each current time point, compute a current filtered data sample ($y_m$) of the filtered pressure signal ($y$) as a function of a preceding state vector ($Z^*$) of the digital filter (60) and a current data sample ($p_m$) in the pressure signal ($p$), and compute a current state vector ($Z_m$) of the digital filter (60) as a function of the preceding state vector ($Z^*$), the current data sample ($p_m$) and, optionally, the current filtered data sample ($y_m$);
detect or predict presence of a disturbance in the pressure signal ($p$);
selectively modify the preceding state vector ($Z^*$) of the digital filter (60) at a selected time point ($t_2$) subsequent to the disturbance in the pressure signal ($p$), so as to suppress influence of the disturbance on the filtered pressure signal ($y$); and
detect and process signal components of physiological origin in the filtered pressure signal ($y$).

**2.** The signal filtering device of claim 1, wherein said selectively modifying of the preceding state vector ($Z^*$) comprises replacing the preceding state vector ($Z^*$) of the digital filter (60) at the selected time point ($t_2$) by a reconfiguration state vector ($Z'$).

**3.** The signal filtering device of claim 2, wherein the processor (11) is further caused to: obtain the reconfiguration state vector ($Z'$) to match a working point of the medical apparatus (1) at the selected time point ($t_2$).

**4.** The signal filtering device of claim 3, wherein the processor (11) is further caused to: acquire, from the data storage memory (12), at least one state vector ([$Z$]) for the working point of the medical apparatus (1) at the selected time point ($t_2$), and obtain the reconfiguration state vector ($Z'$) as a function of the at least one state vector ([$Z$]).

**5.** The signal filtering method device of claim 4, wherein the data storage memory (12) stores a database (12A) that comprises state vectors for a plurality of different working points of the medical apparatus (1), and wherein the processor (11) is caused to select said at least one state vector ([$Z$]) among the state vectors stored in the database (12A) based on the working point of the medical apparatus (1) at the selected time point ($t_2$).

**6.** The signal filtering device of claim 5, wherein the processor (11) is further caused to: populate at least part of the database (12A) during operation of the digital filter (60) prior to the disturbance, by storing one or more of the preceding state vectors ($Z^*$) in association with a respective current working point of the medical apparatus (1).

**7.** The signal filtering device of claim 4, wherein the processor (11) is further caused to: selectively store, when detecting or predicting the presence of the disturbance in the pressure signal ($p$), a preceding state vector ($Z^*$) at a first time point ($t_1$), wherein the at least one state vector ([$Z$]) is acquired to comprise the preceding state vector ($Z^*$) at the first time point ($t_1$).

**8.** The signal filtering device of claim 7, wherein the processor (11) is caused to select the selected time point ($t_2$) based on the first time point ($t_1$).

**9.** The signal filtering device of claim 7 or 8, wherein the processor (11) is caused to select the selected time point ($t_2$) such that the working point of the medical apparatus (1) at the selected time point ($t_2$) corresponds to the working point of the medical apparatus (1) at the first time point ($t_1$).

**10.** The signal filtering device of any one of claims 3-9, wherein the working point is at least partly given by a phase of the blood pump (4).

**11.** The signal filtering device of claim 10, wherein the blood pump (4) operates in a sequence of pulse cycles (R), each pulse cycle (R) resulting in at least one pulsation in the pressure signal (p), and wherein the phase corresponds to a location within the pulse cycle (R).

**12.** The signal filtering device of claim 11, wherein the processor (11) is further caused to: obtain the reconfiguration state vector ($Z'$) associated with a selected location within the pulse cycle (R); determine the selected time point ($t_2$), based on a phase signal ($\theta$) indicative of the phase of the blood pump (4), to correspond to the selected location; and set the preceding state vector ($Z^*$) at the selected time point ($t_2$) to the reconfiguration state vector ($Z'$).

**13.** The signal filtering device of any one of claims 10-12, wherein the working point is further given by at least one of a current operating frequency ($\omega$) of the blood pump (4), an average fluid pressure ($P$) in the medical apparatus (1), and an amplitude of pressure variations in the pressure signal ($p$).

**14.** The signal filtering device of claim 1, wherein the processor (11) is further caused to: determine, based on a phase signal ($\theta$) indicative of a phase of the blood pump (4) in the medical apparatus (1), a first phase value at a first time point ($t_1$) preceding the disturbance; store, in the data storage memory (12), the current state vector ($Z_m$) computed at the first time point ($t_1$); obtain a selected phase value as a function of the first phase value; determine the selected time point ($t_2$), based on the phase signal ($\theta$), to correspond to the selected phase value; acquire, from the data storage memory (12), a reconfiguration state vector (Z') as a function of the current state vector ($Z_m$) computed at the first time point ($t_1$); and set the preceding state vector ($Z'$) at the selected time point ($t_2$) to the reconfiguration state vector ($Z'$).

**15.** The signal filtering device of any one of claims 10-14, wherein the phase corresponds to a stroke position of the blood pump (4).

**16.** The signal filtering device of any one of claims 10-15, wherein the blood pump (4) is a peristaltic pump comprising a rotation element (20, 21) for engaging a tube segment (22), and wherein the phase corresponds to an angular position of the rotation element (20, 21).

**17.** The signal filtering device of any one of the preceding claims, wherein the processor (11) is caused to operate the digital filter (60), when generating the current state vector ($Z_m$) to modify the current data sample ($p_m$) by a first set of filter coefficients ($b_2$, ... $b_n$) and, optionally, to modify the current filtered data sample ($y_m$) by a second set of filter coefficients ($a_2$, ... $a_n$).

**18.** The signal filtering device of any one of the preceding claims, wherein the current state vector ($Z_m$) comprises a predefined number of state values ($z_1$, ... $z_{n-1}$) associated with the current time point, wherein the processor (11) is caused to operate the digital filter (60) to compute the state values as:

$$z_1 = b_2 \cdot p_m + z_2^* - a_2 \cdot y_m$$
$$\vdots$$
$$z_{n-2} = b_{n-1} \cdot p_m + z_{n-1}^* - a_{n-1} \cdot y_m$$
$$z_{n-1} = b_n \cdot p_m - a_n \cdot y_m$$

wherein $z^*_2$, ... $z^*_{n-1}$ are state values of the preceding state vector *(Z*)*, $p_m$ is the current data sample, $y_m$ is the current filtered data sample, and $a_2$,... $a_n$ and $b_2$,... $b_n$ are filter coefficients, wherein $a_2$, ... $a_n$ may be set to zero.

**19.** The signal filtering device of any one of the preceding claims, wherein the processor (11) is caused to operate the digital filter (60) to compute the current filtered data sample ($y_m$) as a function of a state value ($z^*_1$) of the preceding state vector (Z*), and the current data sample (__) modified by a filter coefficient ($b_1$).

**20.** The signal filtering device of claim 19, wherein the processor (11) is caused to operate the digital filter (60) to compute the filtered data sample as:

$$Y_m = b_1 * p_m + z^*_1$$

wherein $b_1$ is said filter coefficient, $p_m$ is the current data sample, and $z^*_1$ is said state value of the preceding state vector (Z*).

**21.** The signal filtering device of any one of the preceding claims, wherein the processor (11) is caused to operate the digital filter (60) to compute the current state vector ($Z_m$) for each current data sample ($p_m$) in the pressure signal ($p$).

**22.** The signal filtering device of any one of the preceding claims, wherein the processor (11) is further caused to: stop the operation of the digital filter (60) during at least part of the predicted or detected disturbance in the pressure signal ($p$).

**23.** The signal filtering device of any preceding claim, wherein the signal components are pressure pulsations originating from one or more physiological pulse generators in the human or animal subject.

**24.** The signal filtering device of any preceding claim, wherein the pressure signal is obtained from a pressure sensor (6a) on a downstream side of the blood pump (4) in the extracorporeal blood flow circuit (1), and wherein the signal filtering device is configured to detect that the access device (2")for blood reintroduction comes loose from the dedicated vascular access (3) by identifying an absence of heart or breathing pulses in the filtered pressure signal (*y*).

**25.** A medical system, comprising:

a medical apparatus (1) for extracorporeal blood processing, in which blood is taken out of a human or animal subject, processed and then reintroduced into the human or animal subject by means of an extracorporeal blood flow circuit (1), said extracorporeal blood flow circuit (1) comprising a blood pump (4) for circulating the blood through the extracorporeal blood flow circuit (1), an access device (2') for blood withdrawal and an access device (2") for blood reintroduction, said access device (2') for blood withdrawal and said access device (2") for blood reintroduction being inserted into a dedicated vascular access of the human or animal subject;
a pressor sensor (6a; 6b) arranged to be responsive to fluid pressure in the medical apparatus; and
a signal filtering device as set forth in any one of claims 1 - 24, wherein the signal filtering device is connected to the pressure sensor (6a; 6b) and is configured to obtain the pressure signal (*p*) from the pressure sensor (6a; 6b).

## Patentansprüche

**1.** Signalfiltervorrichtung, die einen Prozessor (11) und einen Datenspeicherungsspeicher (12) umfasst, der Computeranweisungen speichert, die, wenn sie durch den Prozessor (11) ausgeführt werden, den Prozessor (11) veranlassen zum:

Erhalten eines Drucksignals (p), das eine Zeitsequenz von Datenabtastungen umfasst, die Fluiddruck in einem medizinischen Gerät (1) zur extrakorporalen Blutverarbeitung repräsentieren, wobei Blut aus einem menschlichen oder tierischen Subjekt entnommen, mittels eines extrakorporalen Blutströmungskreislaufs (1) verarbeitet und dann wieder in das menschliche oder tierische Subjekt eingeführt wird, wobei der extrakorporale Blutströmungskreislauf (1) eine Blutpumpe (4) zum Zirkulieren des Blutes durch den extrakorporalen Blutströmungskreislauf (1), eine Zugangsvorrichtung (2') zur Blutentnahme und eine Zugangsvorrichtung (2") zur Blutwiedereinführung umfasst, wobei die Zugangsvorrichtung (2') zur Blutentnahme und die Zugangsvorrichtung (2") zur Blutwiedereinführung in einen dedizierten Gefäßzugang des menschlichen oder tierischen Subjekts eingeführt werden;
Betreiben eines digitalen Filters (60) an dem Drucksignal (p), um ein gefiltertes Drucksignal (y) zu produzieren, in dem von der Blutpumpe (4) stammende Druckpulse entfernt oder unterdrückt werden, wobei das digitale Filter (60) dazu ausgelegt ist, zu jedem aktuellen Zeitpunkt eine aktuelle gefilterte Datenabtastung ($y_m$) des gefilterten Drucksignals (y) als eine Funktion eines vorhergehenden Zustandsvektors (Z*) des digitalen Filters (60) und eine aktuelle Datenabtastung ($p_m$) in dem Drucksignal (p) zu berechnen, und einen aktuellen Zustandsvektor ($Z_m$) des digitalen Filters (60) als eine Funktion des vorhergehenden Zustandsvektors (Z*), der aktuellen Datenabtastung ($p_m$) und gegebenenfalls der aktuellen gefilterten Datenabtastung ($y_m$) zu berechnen;
Detektieren oder Vorhersagen des Vorhandenseins einer Störung in dem Drucksignal (p);
selektives Modifizieren des vorhergehenden Zustandsvektors (Z*) des digitalen Filters (60) zu einem ausgewählten Zeitpunkt ($t_2$) nach der Störung in dem Drucksignal (p), um den Einfluss der Störung auf das gefilterte Drucksignal (y) zu unterdrücken; und Detektieren und Verarbeiten von Signalkomponenten physiologischen Ursprungs in dem gefilterten Drucksignal (y).

**2.** Signalfiltervorrichtung nach Anspruch 1, wobei das selektive Modifizieren des vorhergehenden Zustandsvektors (Z*) Ersetzen des vorhergehenden Zustandsvektors (Z*) des digitalen Filters (60) zu dem ausgewählten Zeitpunkt ($t_2$) durch einen Rekonfigurationszustandsvektor (Z') umfasst.

**3.** Signalfiltervorrichtung nach Anspruch 2, wobei der Prozessor (11) ferner zu Folgendem veranlasst wird: Erhalten des Rekonfigurationszustandsvektors (Z'), so dass er zu einem Arbeitspunkt des medizinischen Geräts (1) zu dem ausgewählten Zeitpunkt ($t_2$) passt.

**4.** Signalfiltervorrichtung nach Anspruch 3, wobei der Prozessor (11) ferner veranlasst wird zum: Erlangen mindestens eines Zustandsvektors ([Z]) für den Arbeitspunkt des medizinischen Geräts (1) zu dem ausgewählten Zeitpunkt ($t_2$) aus dem Datenspeicherungsspeicher (12), und Erhalten des Rekonfigurationszustandsvektors (Z') als eine Funktion des mindestens einen Zustandsvektors ([Z]).

**5.** Signalfilterverfahrensvorrichtung nach Anspruch 4, wobei der Datenspeicherungsspeicher (12) eine Datenbank (12A) speichert, die Zustandsvektoren für eine Vielzahl von verschiedenen Arbeitspunkte des medizinischen Geräts (1) umfasst, und wobei veranlasst wird, dass der Prozessor (11) den mindestens einen Zustandsvektor ([Z]) innerhalb der Zustandsvektoren, die in der Datenbank (12A) gespeichert sind, basierend auf dem Arbeitspunkt des medizinischen Geräts (1) zu dem ausgewählten Zeitpunkt ($t_2$) auswählt.

**6.** Signalfiltervorrichtung nach Anspruch 5, wobei der Prozessor (11) ferner veranlasst wird zum: Bestücken mindestens eines Teils der Datenbank (12A) während des Betriebs des digitalen Filters (60) vor der Störung, indem einer oder mehrere der vorhergehenden Zustandsvektoren (Z*) assoziiert mit einem jeweiligen aktuellen Arbeitspunkt des medizinischen Geräts (1) gespeichert werden.

**7.** Signalfiltervorrichtung nach Anspruch 4, wobei der Prozessor (11) ferner veranlasst wird zum: selektiven Speichern eines vorhergehenden Zustandsvektors (Z*) zu einem ersten Zeitpunkt ($t_1$), wenn das Vorhandensein der Störung in dem Drucksignal (p) detektiert oder vorhergesagt wird, wobei der mindestens eine Zustandsvektor ([Z]) so erlangt wird, dass er den vorhergehenden Zustandsvektor (Z*) zu dem ersten Zeitpunkt ($t_1$) umfasst.

**8.** Signalfiltervorrichtung nach Anspruch 7, wobei veranlasst wird, dass der Prozessor (11) den ausgewählten Zeitpunkt ($t_2$) basierend auf dem ersten Zeitpunkt ($t_1$) auswählt.

**9.** Signalfiltervorrichtung nach Anspruch 7 oder 8, wobei veranlasst wird, dass der Prozessor (11) den ausgewählten Zeitpunkt ($t_2$) so auswählt, dass der Arbeitspunkt des medizinischen Geräts (1) zu dem ausgewählten Zeitpunkt ($t_2$) dem Arbeitspunkt des medizinischen Geräts (1) zu dem ersten Zeitpunkt ($t_1$) entspricht.

**10.** Signalfiltervorrichtung nach einem der Ansprüche 3-9, wobei der Arbeitspunkt zumindest teilweise durch eine Phase der Blutpumpe (4) gegeben ist.

**11.** Signalfiltervorrichtung nach Anspruch 10, wobei die Blutpumpe (4) in einer Sequenz von Pulszyklen (R) arbeitet, wobei jeder Pulszyklus (R) zu mindestens einem Pulsieren in dem Drucksignal (p) führt, und wobei die Phase einer Position innerhalb des Pulszyklus (R) entspricht.

**12.** Signalfiltervorrichtung nach Anspruch 11, wobei der Prozessor (11) ferner veranlasst wird zum: Erhalten des Rekonfigurationszustandsvektors (Z'), der mit einer ausgewählten Position innerhalb des Pulszyklus (R) assoziiert ist; Bestimmen des ausgewählten Zeitpunkts ($t_2$) basierend auf einem Phasensignal ($\theta$), das die Phase der Blutpumpe (4) angibt, so dass er der ausgewählten Position entspricht; und Setzen des vorhergehenden Zustandsvektors (Z*) zu dem ausgewählten Zeitpunkt ($t_2$) auf den Rekonfigurationszustandsvektor (Z').

**13.** Signalfiltervorrichtung nach einem der Ansprüche 10-12, wobei der Arbeitspunkt ferner durch mindestens eines von einer aktuellen Arbeitsfrequenz ($\omega$) der Blutpumpe (4), einem durchschnittlichen Fluiddruck (P) in dem medizinischen Gerät (1) und einer Amplitude von Druckvariationen in dem Drucksignal (p) gegeben ist.

**14.** Signalfiltervorrichtung nach Anspruch 1, wobei der Prozessor (11) ferner veranlasst wird zum: Bestimmen eines ersten Phasenwerts zu einem ersten Zeitpunkt ($t_1$), der der Störung vorausgeht, basierend auf einem Phasensignal ($\theta$), das eine Phase der Blutpumpe (4) in dem medizinischen Gerät (1) angibt; Speichern des aktuellen Zustandsvektors ($Z_m$), der zu dem ersten Zeitpunkt ($t_1$) berechnet wurde, in dem Datenspeicherungsspeicher (12); Erhalten eines ausgewählten Phasenwerts als eine Funktion des ersten Phasenwerts; Bestimmen des ausgewählten Zeitpunkts ($t_2$) basierend auf dem Phasensignal ($\theta$), um dem ausgewählten Phasenwert zu entsprechen; Erlangen eines Rekonfigurationszustandsvektors (Z') als eine Funktion des aktuellen Zustandsvektors ($Z_m$), der zu dem ersten Zeitpunkt ($t_1$) berechnet wurde, aus dem Datenspeicherungsspeicher (12); und Setzen des vorhergehenden Zustandsvektors (Z') an dem ausgewählten Zeitpunkt ($t_2$) auf den Rekonfigurationszustandsvektor (Z').

**15.** Signalfiltervorrichtung nach einem der Ansprüche 10-14, wobei die Phase einer Hubposition der Blutpumpe (4) entspricht.

**16.** Signalfiltervorrichtung nach einem der Ansprüche 10-15, wobei die Blutpumpe (4) eine peristaltische Pumpe ist, die ein Rotationselement (20, 21) zum Eingriff in ein Rohrsegment (22) umfasst, und wobei die Phase einer Winkelposition des Rotationselements (20, 21) entspricht.

**17.** Signalfiltervorrichtung nach einem der vorhergehenden Ansprüche, wobei veranlasst wird, dass der Prozessor (11) das digitale Filter (60) betreibt, wenn der aktuelle Zustandsvektor ($Z_m$) generiert wird, um die aktuelle Datenabtastung ($p_m$) durch einen ersten Satz von Filterkoeffizienten ($b_2$, ... $b_n$) zu modifizieren und gegebenenfalls die aktuelle gefilterte Datenabtastung ($y_m$) durch einen zweiten Satz von Filterkoeffizienten ($a_2$, ... $a_n$) zu modifizieren.

**18.** Signalfiltervorrichtung nach einem der vorhergehenden Ansprüche, wobei der aktuelle Zustandsvektor ($Z_m$) eine vordefinierte Anzahl von Zustandswerten ($z_1$, ... $z_{n-1}$) umfasst, die mit dem aktuellen Zeitpunkt assoziiert sind, wobei veranlasst wird, dass der Prozessor (11) das digitale Filter (60) betreibt, um die Zustandswerte zu berechnen als:

$$\begin{aligned} z_1 &= b_2 \cdot p_m + z_2^* - a_2 \cdot y_m \\ &\vdots \\ z_{n-2} &= b_{n-1} \cdot p_m + z_{n-1}^* - a_{n-1} \cdot y_m \\ z_{n-1} &= b_n \cdot p_m - a_n \cdot y_m \end{aligned}$$

wobei $z^*_2$,... $z^*_{n-1}$ Zustandswerte des vorhergehenden Zustandsvektors (Z*) sind, $p_m$ die aktuelle Datenabtastung ist, $y_m$ die aktuelle gefilterte Datenabtastung ist, und $a_2$,... $a_n$ und $b_2$,... $b_n$ Filterkoeffizienten sind, wobei $a_2$, ... $a_n$ auf Null gesetzt werden können.

**19.** Signalfiltervorrichtung nach einem der vorhergehenden Ansprüche, wobei veranlasst wird, dass der Prozessor (11) das digitale Filter (60) betreibt, um die aktuelle gefilterte Datenabtastung ($y_m$) als eine Funktion eines Zustandswerts ($z^*_1$) des vorhergehenden Zustandsvektors (Z*) und der aktuellen Datenabtastung (__), modifiziert durch einen Filterkoeffizienten ($b_1$), zu berechnen.

**20.** Signalfiltervorrichtung nach Anspruch 19, wobei veranlasst wird, dass der Prozessor (11) das digitale Filter (60) betreibt, um die gefilterte Datenabtastung zu berechnen als:

$$Y_m = b_1 * p_m + z^*_1$$

wobei $b_1$ der Filterkoeffizient ist, $p_m$ die aktuelle Datenabtastung ist, und $z^*_1$ der Zustandswert des vorhergehenden Zustandsvektors (Z*) ist.

**21.** Signalfiltervorrichtung nach einem der vorhergehenden Ansprüche, wobei veranlasst wird, dass der Prozessor (11) das digitale Filter (60) betreibt, um den aktuellen Zustandsvektor ($Z_m$) für jede aktuelle Datenabtastung ($p_m$) in dem Drucksignal (p) zu berechnen.

**22.** Signalfiltervorrichtung nach einem der vorhergehenden Ansprüche, wobei der Prozessor (11) ferner veranlasst wird zum: Stoppen des Betriebs des digitalen Filters (60) während mindestens eines Teils der vorhergesagten oder detektierten Störung in dem Drucksignal (p).

**23.** Signalfiltervorrichtung nach einem der vorhergehenden Ansprüche, wobei die Signalkomponenten Druckpulsierungen sind, die von einem oder mehreren physiologischen Pulsgeneratoren in dem menschlichen oder tierischen Subjekt stammen.

**24.** Signalfiltervorrichtung nach einem der vorhergehenden Ansprüche, wobei das Drucksignal von einem Drucksensor (6a) auf einer stromabwärtigen Seite der Blutpumpe (4) in dem extrakorporalen Blutströmungskreislauf (1) erhalten wird, und wobei die Signalfiltervorrichtung dazu ausgelegt ist, zu detektieren, dass sich die Zugangsvorrichtung (2") zur Blutwiedereinführung von dem dedizierten Gefäßzugang (3) löst, indem eine Abwesenheit von Herz- oder Atempulsen in dem gefilterten Drucksignal (y) identifiziert wird.

**25.** Medizinisches System, umfassend:

ein medizinisches Gerät (1) zur extrakorporalen Blutverarbeitung, wobei Blut aus einem menschlichen oder tierischen Subjekt entnommen, mittels eines extrakorporalen Blutströmungskreislaufs (1) verarbeitet und anschließend wieder in das menschliche oder tierische Subjekt eingeführt wird, wobei der extrakorporale Blutströmungskreislauf (1) eine Blutpumpe (4) zum Zirkulieren des Blutes durch den extrakorporalen Blutströmungskreislauf (1), eine Zugangsvorrichtung (2') zur Blutentnahme und eine Zugangsvorrichtung (2") zur Blutwiedereinführung umfasst, wobei die Zugangsvorrichtung (2') zur Blutentnahme und die Zugangsvorrichtung (2") zur Blutwiedereinführung in einen dedizierten Gefäßzugang des menschlichen oder tierischen Subjekts eingeführt werden;
einen Drucksensor (6a; 6b), der so angeordnet ist, dass er auf Fluiddruck in der medizinischen Vorrichtung anspricht; und
eine Signalfiltervorrichtung nach einem der Ansprüche 1-24, wobei die Signalfiltervorrichtung mit dem Drucksensor (6a; 6b) verbunden ist und dazu ausgelegt ist, das Drucksignal (p) von dem Drucksensor (6a; 6b) zu erhalten.

## Revendications

**1.** Dispositif de filtrage de signaux, comprenant un processeur (11) et une mémoire de stockage de données (12) stockant des instructions informatiques qui, lorsqu'elles sont exécutées par le processeur (11), amènent le processeur (11) à :

obtenir un signal de pression (p) comprenant une séquence temporelle d'échantillons de données représentant la pression d'un fluide dans un appareil médical (1) de traitement extracorporel du sang, dans lequel le sang est prélevé sur un sujet humain ou animal, traité puis réintroduit dans le sujet humain ou animal au moyen d'un circuit de circulation sanguine extracorporel (1), ledit circuit de circulation sanguine extracorporel (1) comprenant une pompe à sang (4) pour faire circuler le sang à travers le circuit de circulation sanguine extracorporel (1), un dispositif d'accès (2') pour prélèvement sanguin et un dispositif d'accès (2") pour réintroduction sanguine, ledit dispositif d'accès (2') pour prélèvement sanguin et ledit dispositif d'accès (2") pour réintroduction sanguine étant insérés dans un accès vasculaire dédié du sujet humain ou animal ;
appliquer un filtre numérique (60) sur le signal de pression (p) pour produire un signal de pression filtré (y) dans lequel des impulsions de pression provenant de la pompe à sang (4) sont retirées ou atténuées, le filtre numérique (60) étant configuré pour, à chaque instant courant, calculer un échantillon de données filtré courant ($y_m$) du signal de pression filtré (y) en fonction d'un vecteur d'état précédent (Z*) du filtre numérique (60) et d'un échantillon de données courant ($p_m$) dans le signal de pression (p), et calculer un vecteur d'état courant ($Z_m$) du filtre numérique (60) en fonction du vecteur d'état précédent (Z*), de l'échantillon de données courant ($p_m$) et, éventuellement, de l'échantillon de données filtré courant ($y_m$) ;
détecter ou prédire la présence d'une perturbation dans le signal de pression (p) ;
modifier de manière sélective le vecteur d'état précédent (Z*) du filtre numérique (60) à un point temporel sélectionné ($t_2$) suivant la perturbation du signal de pression (p), de manière à supprimer l'influence de la perturbation sur le signal de pression filtré (y) ; et détecter et traiter des composantes du signal d'origine physiologique dans le signal de pression filtré (y).

**2.** Dispositif de filtrage de signaux selon la revendication 1, ladite modification sélective du vecteur d'état précédent (Z*) comprenant le remplacement du vecteur d'état précédent (Z*) du filtre numérique (60) au point temporel sélectionné ($t_2$) par un vecteur d'état de reconfiguration (Z').

**3.** Dispositif de filtrage de signaux selon la revendication 2, le processeur (11) étant en outre amené à : obtenir le vecteur d'état de reconfiguration (Z') pour correspondre à un point de travail de l'appareil médical (1) au point temporel sélectionné ($t_2$).

**4.** Dispositif de filtrage de signaux selon la revendication 3, le processeur (11) étant en outre amené à : acquérir, à partir de la mémoire de stockage de données (12), au moins un vecteur d'état ([Z]) pour le point de travail de l'appareil médical (1) au point temporel sélectionné ($t_2$), et obtenir le vecteur d'état de reconfiguration (Z') en fonction de l'au moins un vecteur d'état ([Z]).

**5.** Dispositif de procédé de filtrage de signaux selon la revendication 4, la mémoire de stockage de données (12) stockant une base de données (12A) qui comprend des vecteurs d'état pour une pluralité de points de travail différents de l'appareil médical (1), et le processeur (11) étant amené à sélectionner ledit au moins un vecteur d'état ([Z]) parmi les vecteurs d'état stockés dans la base de données (12A) sur la base du point de travail de l'appareil médical (1) au point temporel sélectionné ($t_2$).

**6.** Dispositif de filtrage de signaux selon la revendication 5, le processeur (11) étant en outre amené à : renseigner au moins une partie de la base de données (12A) pendant le fonctionnement du filtre numérique (60) avant la perturbation, en stockant un ou plusieurs des vecteurs d'état précédents (Z*) en association avec un point de travail courant respectif de l'appareil médical (1).

**7.** Dispositif de filtrage de signaux selon la revendication 4, le processeur (11) étant en outre amené à : stocker sélectivement, lors de la détection ou de la prédiction de la présence de la perturbation dans le signal de pression (p), un vecteur d'état précédent (Z*) à un premier point temporel ($t_1$), l'au moins un vecteur d'état ([Z]) étant acquis pour comprendre le vecteur d'état précédent (Z*) au premier point temporel ($t_1$).

**8.** Dispositif de filtrage de signaux selon la revendication 7, le processeur (11) étant amené à sélectionner le point temporel sélectionné ($t_2$) sur la base du premier point temporel ($t_1$).

**9.** Dispositif de filtrage de signaux selon la revendication 7 ou 8, le processeur (11) étant amené à sélectionner le point temporel sélectionné ($t_2$) de telle sorte que le point de travail de l'appareil médical (1) au point temporel sélectionné ($t_2$) corresponde au point de travail de l'appareil médical (1) au premier point temporel ($t_1$) .

**10.** Dispositif de filtrage de signaux selon l'une quelconque des revendications 3 à 9, le point de travail étant au moins partiellement donné par une phase de la pompe à sang (4).

**11.** Dispositif de filtrage de signaux selon la revendication 10, la pompe à sang (4) fonctionnant dans une séquence de cycles d'impulsions (R), chaque cycle d'impulsions (R) résultant en au moins une pulsation dans le signal de pression (p), et la phase correspondant à un emplacement à l'intérieur du cycle d'impulsions (R).

**12.** Dispositif de filtrage de signaux selon la revendication 11, le processeur (11) étant en outre amené à : obtenir le vecteur d'état de reconfiguration (Z') associé à un emplacement sélectionné dans le cycle d'impulsions (R) ; déterminer le point temporel sélectionné ($t_2$), sur la base d'un signal de phase ($\theta$) indicatif de la phase de la pompe à sang (4), pour correspondre à l'emplacement sélectionné ; et régler le vecteur d'état précédent (Z*) au point temporel sélectionné ($t_2$) sur le vecteur d'état de reconfiguration (Z').

**13.** Dispositif de filtrage de signaux selon l'une quelconque des revendications 10 à 12, le point de travail étant en outre donné par au moins l'un d'une fréquence de fonctionnement courante ($\omega$) de la pompe à sang (4), d'une pression de fluide moyenne (P) dans l'appareil médical (1), et d'une amplitude de variations de pression dans le signal de pression (p).

**14.** Dispositif de filtrage de signaux selon la revendication 1, le processeur (11) étant en outre amené à : déterminer, sur la base d'un signal de phase ($\theta$) indicatif d'une phase de la pompe à sang (4) dans l'appareil médical (1), une première valeur de phase à un premier point temporel ($t_1$) précédant la perturbation ; stocker, dans la mémoire de stockage de données (12), le vecteur d'état courant ($Z_m$) calculé au premier point temporel ($t_1$) ; obtenir une valeur de phase sélectionnée en fonction de la première valeur de phase ; déterminer le point temporel sélectionné ($t_2$), sur la base du signal de phase ($\theta$), pour correspondre à la valeur de phase sélectionnée ; acquérir, à partir de la mémoire de stockage de données (12), un vecteur d'état de reconfiguration (Z') en fonction du vecteur d'état courant ($Z_m$) calculé au premier point temporel ($t_1$) ; et fixer le vecteur d'état précédent (Z') au point temporel sélectionné ($t_2$) au vecteur d'état de reconfiguration (Z').

**15.** Dispositif de filtrage de signaux selon l'une quelconque des revendications 10 à 14, la phase correspondant à une position de course de la pompe à sang (4).

**16.** Dispositif de filtrage de signaux selon l'une quelconque des revendications 10 à 15, la pompe à sang (4) étant une pompe péristaltique comprenant un élément de rotation (20, 21) pour engager un segment de tube (22), et la phase correspondant à une position angulaire de l'élément de rotation (20, 21).

**17.** Dispositif de filtrage de signaux selon l'une quelconque des revendications précédentes, le processeur (11) étant amené à faire fonctionner le filtre numérique (60), lors de la génération du vecteur d'état courant ($Z_m$), pour modifier l'échantillon de données courant ($p_m$) par un premier ensemble de coefficients de filtrage ($b_2$, ... $b_n$) et, éventuellement, pour modifier l'échantillon de données filtré courant ($y_m$) par un second ensemble de coefficients de filtrage ($a_2$, ... $a_n$) .

**18.** Dispositif de filtrage de signaux selon l'une quelconque des revendications précédentes, le vecteur d'état courant ($Z_m$) comprenant un nombre prédéfini de valeurs d'état ($z_1$, ... $z_{n-1}$) associé au point temporel courant, le processeur (11) étant amené à faire fonctionner le filtre numérique (60) pour calculer les valeurs d'état comme suit :

$$z_1 = b_2 \cdot p_m + z_2^* - a_2 \cdot y_m$$
$$\vdots$$
$$z_{n-2} = b_{n-1} \cdot p_m + z_{n-1}^* - a_{n-1} \cdot y_m$$
$$z_{n-1} = b_n \cdot p_m - a_n \cdot y_m$$

$z^*_2$,... $z^*_{n-1}$ étant des valeurs d'état du vecteur d'état précédent (Z*), $p_m$ étant l'échantillon de données courant, $y_m$ étant l'échantillon de données filtré courant, et $a_2$,... $a_n$ et $b_2$,... $b_n$ étant des coefficients de filtre, $a_2$, ... $a_n$ pouvant être défini sur zéro.

**19.** Dispositif de filtrage de signaux selon l'une quelconque des revendications précédentes, le processeur (11) étant amené à faire fonctionner le filtre numérique (60) pour calculer l'échantillon de données filtré courant ($y_m$) en fonction d'une valeur d'état ($z^*_1$) du vecteur d'état précédent (Z*), et l'échantillon de données courant (_) modifié par un coefficient de filtre ($b_1$).

**20.** Dispositif de filtrage de signaux selon la revendication 19, le processeur (11) étant amené à faire fonctionner le filtre numérique (60) pour calculer l'échantillon de données filtré comme suit :

$$Y_m = b_1 * p_m + z^*_1$$

$b_1$ étant ledit coefficient de filtre, $p_m$ étant l'échantillon de données courant et $z^*_1$ étant ladite valeur d'état du vecteur d'état précédent (Z*).

**21.** Dispositif de filtrage de signaux selon l'une quelconque des revendications précédentes, le processeur (11) étant amené à faire fonctionner le filtre numérique (60) pour calculer le vecteur d'état courant ($Z_m$) pour chaque échantillon de données courant ($p_m$) dans le signal de pression (p).

**22.** Dispositif de filtrage de signaux selon l'une quelconque des revendications précédentes, le processeur (11) étant en outre amené à : arrêter le fonctionnement du filtre numérique (60) pendant au moins une partie de la perturbation prédite ou détectée dans le signal de pression (p).

**23.** Dispositif de filtrage de signaux selon l'une quelconque des revendications précédentes, les composantes de signal étant des pulsations de pression provenant d'un ou plusieurs générateurs d'impulsions physiologiques chez le sujet humain ou animal.

**24.** Dispositif de filtrage de signaux selon l'une quelconque des revendications précédentes, le signal de pression étant obtenu à partir d'un capteur de pression (6a) sur un côté aval de la pompe à sang (4) dans le circuit de circulation sanguine extracorporelle (1), et le dispositif de filtrage de signaux étant configuré pour détecter que le dispositif d'accès (2") pour la réintroduction sanguine se détache de l'accès vasculaire dédié (3) en identifiant une absence d'impulsions cardiaques ou respiratoires dans le signal de pression filtré (y).

**25.** Système médical, comprenant :

un appareil médical (1) pour le traitement extracorporel du sang, dans lequel le sang est prélevé sur un sujet humain ou animal, traité puis réintroduit dans le sujet humain ou animal au moyen d'un circuit de circulation sanguine extracorporel (1), ledit circuit de circulation sanguine extracorporel (1) comprenant une pompe à sang (4) pour faire circuler le sang à travers le circuit de circulation sanguine extracorporel (1), un dispositif d'accès (2') pour le prélèvement sanguin et un dispositif d'accès (2") pour la réintroduction sanguine, ledit dispositif d'accès (2') pour le prélèvement sanguin et ledit dispositif d'accès (2") pour la réintroduction sanguine étant insérés dans un accès vasculaire dédié du sujet humain ou animal ;
un capteur de pression (6a ; 6b) agencé pour réagir à la pression du fluide dans l'appareil médical ; et
un dispositif de filtrage de signaux selon l'une quelconque des revendications 1 à 24, le dispositif de filtrage de signaux étant relié au capteur de pression (6a ; 6b) et étant configuré pour obtenir le signal de pression (p) provenant du capteur de pression (6a ; 6b).

100
PH
11
9
12
10
θ
y
p
6b
5
2"
2'
3
6a
4
8
1

FIG. 1

21
4
20
22
21
8
θ

FIG. 2

p
θm
Pressure
pm
Time
R
R
R
R

FIG. 3

T
1
0
40
41
$\omega_1$
$\omega_2$
T
42
1
0
$0.5\omega$
$\omega$
$1.5\omega$
$2\omega$
$2.5\omega$
$p_m$
$b_n$
$b_3$
$b_2$
$b_1$
$a_n$
$a_3$
$a_2$
$z_{n-1}$
$z_2$
$z_1$
$Z^{-1}$
$y_m$
54
52
53
55
50
51
Time
123
122
121
120
$y$
$z_1$
$z_2$
$z_{n-2}$
$z_{n-1}$
$\mathbf{Z}_{123}$
$\mathbf{Z}_{122}$
$\mathbf{Z}_{121}$
$\mathbf{Z}_{120}$

FIG. 4A

FIG. 4B

FIG. 5A

FIG. 5B

θ
ω
P̄
600
12
62
D
[Z]
Z′
C
60
64
p
y

FIG. 6

1
y
10
9
p
2"
6a
11
12
7
8
4
θ

FIG. 11

SIGNAL FILTERING
700
OBTAIN PRESSURE SIGNAL, $p$
701
INPUT INITIAL STATE VECTOR, $Z_s^*$
702
INPUT PRESSURE SAMPLE, $p_m$
703
COMPUTE FILTERED SAMPLE, $y_m$, AS A FUNCTION OF $p_m$ AND $Z^*$
704
COMPUTE CURRENT STATE VECTOR, $Z_m$, AS A FUNCTION OF $Z^*$, $p_m$ AND $y_m$
705
706
DISTURBANCE?
Y
N
708
STOP FILTERING
709
DISTURBANCE ENDED?
N
Y
710
OBTAIN $Z'$ FOR SELECTED WORKING POINT
711
SET $Z^* = Z'$ AT SELECTED WORKING POINT
712
RESTART FILTERING
707
NEXT PRESSURE SAMPLE

FIG. 7

12
$\boldsymbol{Z}^*$
62
$[\boldsymbol{Z}] = \boldsymbol{Z}^*$
$\boldsymbol{Z}' = \boldsymbol{Z}^*$

FIG. 8A

12
$\theta_t$
$\boldsymbol{Z}_i : \theta_i$
62
$[\boldsymbol{Z}]$
$\boldsymbol{Z}' = f([\boldsymbol{Z}])$

FIG. 8B

12A

| $\bar{P}$ | $\omega$ | $\theta$ | $\boldsymbol{Z}$ |
|---|---|---|---|
| $\bar{P}_1$ | $\omega_1$ | $\theta_1$ | $\boldsymbol{Z}_1$ |
| $\bar{P}_1$ | $\omega_1$ | $\theta_2$ | $\boldsymbol{Z}_2$ |
| ⋮ | ⋮ | ⋮ | ⋮ |
| $\bar{P}_1$ | $\omega_1$ | $\theta_q$ | $\boldsymbol{Z}_q$ |
| $\bar{P}_2$ | $\omega_1$ | $\theta_1$ | $\boldsymbol{Z}_{q+1}$ |
| $\bar{P}_2$ | $\omega_1$ | $\theta_2$ | $\boldsymbol{Z}_{q+2}$ |
| ⋮ | ⋮ | ⋮ | ⋮ |
| $\bar{P}_2$ | $\omega_1$ | $\theta_q$ | $\boldsymbol{Z}_{2q}$ |
| $\bar{P}_1$ | $\omega_2$ | $\theta_1$ | $\boldsymbol{Z}_{2q+1}$ |
| $\bar{P}_1$ | $\omega_2$ | $\theta_2$ | $\boldsymbol{Z}_{2q+2}$ |
| ⋮ | ⋮ | ⋮ | ⋮ |
| $\bar{P}_1$ | $\omega_2$ | $\theta_q$ | $\boldsymbol{Z}_{3q}$ |
| ⋮ | ⋮ | ⋮ | ⋮ |

FIG. 8C

$t_1$
$t_P$
$t_S$
$t_2$
Time
$p$
$\theta_{t1}$
$\theta_{t2}$
$\theta$
$Z$
$Z_m$
$Z'$
$N$
$y$
R
R
R
R
R
R


FIG. 9

30
25
20
15
10
5
0
-5
-10
-15
Pressure [au]
0
1
2
3
4
5
6
7
8
9
10
Time [s]
y
y'

FIG. 10A

20
15
10
5
0
-5
-10
Pressure [au]
0
5
10
15
20
25
30
35
40
45
Time [s]
y'
y

FIG. 10B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9710013 A
- US 20050010118 A
- WO 2009156174 A
- WO 2010149726 A
- US 20100234786 A
- WO 2011080189 A
- WO 2011080190 A
- WO 2011080191 A
- WO 2011080194 A
- WO 2014147028 A
- WO 2011080188 A
- WO 2009156175 A
- WO 2013000777 A
- WO 2016206949 A
- WO 2014009111 A
- WO 2015032948 A
- US 20140199193 A
- WO 2014035947 A
- AU 2014250616
- WO 2012170119 A